(19) **Europäisches Patentamt / European Patent Office / Office européen des brevets**

(11) **EP 3 156 792 B1**

(12) **FASCICULE DE BREVET EUROPEEN**

(45) Date de publication et mention de la délivrance du brevet:
**26.01.2022 Bulletin 2022/04**

(21) Numéro de dépôt: **16193440.1**

(22) Date de dépôt: **12.10.2016**

(51) Classification Internationale des Brevets (IPC):
**G01N 31/16** *(2006.01)*     *G01N 33/18* *(2006.01)*

(52) Classification Coopérative des Brevets (CPC):
**G01N 31/16;** G01N 31/164; G01N 33/188

(54) **PROCÉDÉ D'ESTIMATION DE LA CONCENTRATION DE COMPOSÉS D'INTÉRÊT DE SUBSTRAT OU DE DIGESTAT DE METHANISEUR**

VERFAHREN ZUM ABSCHÄTZEN DER KONZENTRATION VON BESTIMMTEN SUBSTRAT- ODER GÄRGUTBESTANDTEILEN EINER BIOGASANLAGE

METHOD FOR ESTIMATING THE CONCENTRATION OF COMPOUNDS OF INTEREST IN A DIGESTER SUBSTRATE OR DIGESTATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.10.2015 FR 1559767**

(43) Date de publication de la demande:
**19.04.2017 Bulletin 2017/16**

(73) Titulaires:
- **BIOENTECH**
  **35400 Saint-Malo (FR)**
- **Institut national de recherche pour l'agriculture, l'alimentation et l'environnement**
  **75007 Paris (FR)**

(72) Inventeurs:
- **LATRILLE, Eric**
  **11110 SALLES D'AUDE (FR)**
- **STEYER, Jean-Philippe**
  **11200 NEVIAN (FR)**
- **MIROUX, Jérémie**
  **69780 SAINT PIERRE DE CHANDIEU (FR)**
- **GUIZARD, Guillaume**
  **11590 CUXAC D'AUDE (FR)**
- **LARDON, Laurent**
  **1660 COPENHAGUE (DK)**
- **CHARNIER, Cyrille**
  **78350 JOUY EN JOSAS (FR)**

(74) Mandataire: **Germain Maureau**
  **12, rue Boileau**
  **69006 Lyon (FR)**

(56) Documents cités:
**JP-A- H05 169 100**    **US-A- 5 132 916**
**US-A- 5 640 330**

**Description**

La présente invention concerne une méthode de titrage de composés d'intérêt présents dans des milieux complexes que sont les substrats ou les digestats de méthaniseurs de différentes installations industrielles (par exemple les installations agricoles, les unités collectives ou encore les stations d'épuration des eaux usées) et qui sont utilisés pour l'optimisation et le suivi du fonctionnement des méthaniseurs.

Dans le cadre de la présente invention, par « digestat », on entend le second résidu, autre que le biogaz, qui est généré au cours d'un processus de méthanisation de la matière organique. Il s'agit d'un résidu solide ou liquide pâteux composé d'éléments organiques non dégradés et de minéraux.

La méthanisation (à savoir un procédé de digestion anaérobie) est un processus naturel biologique de dégradation de la matière organique en l'absence d'oxygène. Elle est effectuée dans des installations industrielles que sont les méthaniseurs (ou autrement dit des digesteurs).

Dans la description qui suit, on entendra par « substrat », un substrat qui est introduit en entrée d'un méthaniseur.

Dans le contexte actuel de la méthanisation, l'azote ammoniacal, les acides gras volatils (ci-après abrégé « AGV ») font partie des principaux composés d'intérêt qui sont utilisés pour le suivi de ces procédés de digestion anaérobie. De plus, de par sa capacité à tamponner l'acidification d'un digesteur, le carbone inorganique est aussi un composé d'intérêt dont l'évolution de sa concentration est aussi intéressante à suivre pour avoir une idée précise de ce qui se passe dans le digesteur au cours de son fonctionnement.

Dans le cadre de la présente invention, l'expression « azote ammoniacal » désigne aussi bien les ions ammoniums ($NH_4^+$) que l'ammoniac ($NH_3$).

Les AGV sont produits à partir des monomères issus de l'hydrolyse du substrat introduit à l'entrée du méthaniseur. Cette réaction appelée acidogénèse est souvent la plus rapide. Par conséquent, en cas de surcharge organique, la réaction s'emballe et engendre une accumulation d'AGV, inhibiteurs pour l'acétogénèse et la méthanogénèse. Cette inhibition est d'autant plus contraignante que la dégradation de ces AGV est réalisée lors de l'acétogénèse et de la méthanogénèse. L'accumulation d'AGV dans le réacteur devient rapidement irréversible et peut engendrer l'arrêt du digesteur. Les concentrations inhibitrices varient selon les processus et l'adaptation de la flore. Il est important que les populations méthanogènes et acétogènes se développent avant d'augmenter la charge organique du digesteur. Par exemple, sur un digesteur alimenté en glucose et cellulose, l'inhibition débute avec une concentration en AGV de 4g/L. Sur d'autres procédés, comme la co-digestion de déchets ménagers et de lisier de porc, la concentration inhibitrice d'AGV est relevée à 3g/L.

Cette concentration est aussi grandement dépendante du pH, car la forme acide porte l'inhibition. Par conséquent un second paramètre essentiel à suivre est la concentration en carbone inorganique qui tamponne l'effet d'inhibition et d'acidification des AGV. Le pKa du couple $HCO_3^-/H_2CO_3$ étant à 6,3, la majeure partie du carbone inorganique passera sous forme $H_2CO_3$ avant que l'acidification ne devienne critique, ralentissant fortement celle-ci.

Dans les procédés de digestion anaérobie, l'azote ammoniacal est principalement présent sous forme d'ammonium ($NH_4^+$). Ce composé, quand il n'est pas ionisé ($NH_3$), est toxique pour les micro-organismes car perméable à leur membrane. Il a été montré que lors d'une fermentation anaérobie en condition mésophile, 3g/L d'ammonium suffisait à inhiber 50% de la méthanogénèse. Selon les conditions, la concentration critique d'azote ammoniacal dans un digesteur mésophile varie.

L'azote ammoniacal est donc un paramètre clé du pilotage des procédés de digestion anaérobie. Celui-ci est d'autant plus concentré que le substrat est riche en azote inorganique ou organique (urée ou protéine). Il est ainsi reconnu comme un facteur primordial pour la méthanisation dans des installations agricoles ou la méthanisation des micro-algues.

Ainsi, les AGV, l'azote ammoniacal et le carbone inorganique sont trois composés d'intérêt dont il convient de surveiller scrupuleusement l'évolution de leur concentration lors de l'utilisation des digesteurs afin de s'assurer de leur bon fonctionnement.

Des méthodes analytiques existent aujourd'hui pour doser les AGV, le carbone inorganique et l'azote ammoniacal. Mais aucune de ces méthodes ne permet de quantifier la concentration de ces trois composés d'intérêt selon une analyse fiable et parfaitement adaptée aux milieux complexes tels que ceux détaillés ci-dessus. En effet, les installations mettant en œuvre ces méthodes analytiques sont souvent souséquipées en capteurs adaptés.

Plus précisément, pour analyser les concentrations des composés d'intérêt des digestats de méthaniseur, on connaît des méthodes de titrage par pH-métrie. Il existe peu de capteurs automatisés permettant un titrage automatique et en ligne. A cet égard, on connaît le capteur titrimétrique connu sous la dénomination « ANASENSE » développé par l'INRA (INRA étant l'acronyme pour « Institut National de Recherche Agronomique »). Ce capteur fournit une estimation du bicarbonate et des AGV par titrage acide. L'intérêt principal de ce capteur est qu'il réalise un titrage automatique toutes les 30 minutes sans recourir à une quelconque intervention humaine pendant 30 jours. Lors du titrage suivi par pH-métrie, un volume d'acide est ajouté automatiquement par une pompe. Une analyse du volume titrant est ensuite effectuée en des points clés, c'est-à-dire avant et après le pKa de l'acétate et de l'hydrogénocarbonate, à savoir là où ces composés titrés sont totalement basiques, puis totalement acides. Le titrage dure au total 10 à 15 minutes. Par différence entre ces points, une estimation des AGV et de l'hydrogénocarbonate est obtenue à partir d'une équation théorique et de facteurs établis sur l'expérience. Avec ce capteurtitrimétique, il ne se pose pas le problème du dégazage du fait de son utilisation en atmosphère fermée et d'un temps de titrage rapide.

Cependant, le capteur titrimétrique dénommé « ANASENSE » présente les inconvénients suivants :

- son coût d'exploitation est relativement élevé ;
- il a tendance à s'encrasser ; ce qui nécessite une intervention tous les 30 jours environ ;
- la présence d'autres acides ne peut être prise en compte et perturbe parfois fortement l'analyse ;
- il peut manquer de précision car l'analyse est faite sur un nombre trop restreint de points.

Pour surmonter ce dernier problème évoqué avec le capteur titrimétrique « ANASENSE », un système de titrage automatique s'appuyant sur l'intégralité de la courbe de titrage a été proposé. L'intérêt est de réaliser une analyse plus riche en informations permettant de doser plusieurs espèces. Ce système de titrage automatique dose le phosphate, l'azote ammoniacal, les AGV et le carbone inorganique grâce à l'analyse de la capacité tampon de l'échantillon de digestat. Ce système a été testé sur différentes solutions répondant à des problématiques actuelles de méthanisation telle que les algues ou des mélanges fumiers/lisier.

Certaines études ont montré que sur des échantillons de digestats de méthaniseur non complexes tels que la vinasse, les concentrations en composés inhibiteurs déterminées avec le capteur titrimétrique « ANASENSE » étaient plus fiables que le système de titrage mettant en œuvre l'analyse de la capacité tampon. Par contre, pour des échantillons plus complexes ou en cas de surcharge organique, l'étude de la capacité tampon est plus appropriée. Malgré tout, l'étude de la capacité tampon ne permet pas d'estimer l'ammonium avec une précision supérieure à 20%, et ce du fait de la possible présence d'autres espèces non prises en compte dans le modèle et influant sur le titrage. Cela peut expliquer pourquoi aucune exploitation industrielle n'a été développée sur ce système de titrage automatique mettant en œuvre l'analyse de la capacité tampon.

Enfin, on connaît une méthode analytique développée par le centre fédéral de recherche agricole allemand et qui est dénommée « FOS/TAC » (« FOS » et « TAC » étant les acronymes allemands respectifs de « Flüchtige Organische Säuren » et de « Totales Anorganisches Carbonat » qui se traduisent respectivement par AGV et carbone inorganique total). La méthode « FOS/TAC » est une des méthodes de suivi des unités d'installations agricoles les plus répandues.

La méthode « FOS/TAC » permet un suivi simplifié des installations à temps de séjour long (à savoir plus de 20 jours) et peu instrumentées, typiquement, les installations agricoles. Cette méthode consiste à filtrer grossièrement un échantillon de substrat ou de digestat de méthaniseur, puis à le titrer à l'acide sulfurique dilué. Des modèles ont été mis au point expérimentalement par régression linéaire de la concentration d'AGV en solution en fonction du volume titrant afin de déterminer les quantités d'AGV exprimées en mg d'acétate équivalent par litre de digestat et de carbone inorganique total exprimées en mg de $CaCO_3$ par litre de digestat.

De plus, selon le ratio de ces deux quantités déterminées obtenu, des recommandations sur l'utilisation du digesteur ont été élaborées dans le cadre de cette méthode « FOS/TAC » afin d'en assurer un fonctionnement le plus durable possible sans que ne se posent les problèmes liés au composés inhibiteurs précités.

La méthode « FOS/TAC » est simple de mise en oeuvre et permet à tout utilisateur d'obtenir des informations sur l'état de fonctionnement du digesteur anaérobie. De plus, une version automatisée de cette méthode « FOS/TAC » a été mise au point. Par contre, certaines études ont montré que les modèles utilisés dans le cadre de la méthode « FOS/TAC » fournissent des résultats de concentrations des AGV et du carbone inorganique qui peuvent être erronés.

Le document JP H05 169100 A décrit un procédé de contrôle d'un méthaniseur consistant en l'analyse de données de pH obtenues par un titrage et analysées avec un procédé d'analyse de régression multiple.

Le brevet US 5 640 330 décrit un système qui corrèle des données expérimentales de pH obtenues par titrage à un modèle de titrage. Les données simulées ainsi générées peuvent être utilisées pour prédire l'évolution du pH dans un système donné.

Ainsi, on relève de l'état de l'art concernant la mise en œuvre de méthodes de titrage automatisées que :

- le titrage acide ne suffit pas à doser l'azote ammoniacal ;
- plus le nombre d'inconnues augmente, moins le système est précis ;
- un système de titrage basé sur des points clés est peu modulable et perd en précision en cas de surcharge organique ;
- les capteurs titrimétriques automatisés tels que « ANASENSE » ou les capteurs de titrage mettant en œuvre l'analyse de la capacité tampon sont satisfaisants pour des échantillons non complexes mais perdent en précision pour des échantillons complexes où il est souvent difficile d'estimer le nombre de tampons réels en se concentrant uniquement sur le pH.

Par ailleurs, le développement d'un capteur en ligne totalement automatisé peut engendrer une multiplication du coût de cet outil d'analyse par dix.

La présente invention se propose de pallier les inconvénients mentionnés ci-dessus des capteurs et des méthodes titrimétriques automatisés connus de l'état de l'art.

La présente invention a pour objet un procédé d'estimation de la concentration en composés d'intérêt tels que les AGV, l'azote ammoniaca et le carbone inorganique, présents dans un échantillon d'un digestat de méthaniseur ou d'un substrat qui est introduit en entrée d'un méthaniseur , le suivi de l'évolution de la concentration de ces composés d'intérêt étant utile pour la surveillance du fonctionnement du méthaniseur.

Le procédé d'estimation selon la présente invention peut être utilisé pour optimiser les paramètres du méthaniseur avant sa mise en fonctionnement. C'est pourquoi, ledit procédé d'estimation peut être appliqué à un substrat qui est introduit en entrée d'un méthaniseur.

Le procédé selon l'invention met en œuvre un titrage qui peut être suivi par pH-métrie et conductimétrie. Par ailleurs, un algorithme permettant de simuler ce titrage en fonction des concentrations en composés d'intérêt tels que l'azote ammoniacal, le carbone inorganique et les AGV présents dans l'échantillon de substrat ou de digestat a été mis au point de manière à fournir un modèle de référence d'échantillon théorique du titrage du substrat ou du digestat. Par comparaison entre le modèle de référence d'échantillon théorique et le modèle d'échantillon expérimental déterminé à partir de données expérimentales recueillies au cours du titrage, une estimation des concentrations des composés d'intérêt précités présents dans l'échantillon de substrat ou de digestat est ainsi obtenue.

La présente invention concerne :

- un procédé d'estimation de la concentration de composés d'intérêt choisis parmi le carbone inorganique et les AGV présents dans un échantillon d'un substrat ou d'un digestat d'un méthaniseur selon la revendication 1,
- un procédé d'estimation de la concentration de composés d'intérêt choisis parmi l'azote ammoniacal et les AGV présents dans un échantillon d'un substrat ou d'un digestat d'un méthaniseur selon la revendication 2,

le suivi de l'évolution de la concentration des composés d'intérêt étant utile pour l'optimisation des paramètres du méthaniseur avant sa mise en fonctionnement, puis la surveillance de son fonctionnement.

L'échantillon de substrat ou de digestat peut être un échantillon liquide ou solide. Lorsqu'il s'agit d'un échantillon solide, il est avantageusement dilué dans de l'eau, de préférence de l'eau distillée, avant la mise en œuvre du titrage.

Le procédé d'estimation selon l'invention présente l'avantage de ne pas nécessiter de filtration de l'échantillon de substrat ou de digestat avant le titrage.

Le titrage est un titrage volumétrique consistant à ajouter un volume dit « volume titrant » d'une solution titrante comprenant au moins une espèce titrante dans ledit l'échantillon de substrat ou de digestat de manière à former une solution réactionnelle.

L'espèce titrante peut être :

- une espèce titrante acide, le titrage étant alors un titrage acide, ou
- une espèce titrante basique, ledit titrage étant alors un titrage basique.

Préférentiellement, l'espèce titrante acide est choisie parmi les acides forts. Dans le cadre de la présente invention, on entend par « acide fort », un acide qui, en solution aqueuse, se dissocie totalement en ions $H^+$ et une base très faible dite base conjuguée de l'acide.

De manière avantageuse, les acides forts sont choisis parmi l'acide chlorhydrique, l'acide nitrique, l'acide iodhydrique, l'acide bromhydrique, l'acide perchlorique, l'acide chlorique, l'acide permanganique, l'acide sulfurique et l'acide manganique.

Préférentiellement, l'espèce titrante basique est choisie parmi les bases fortes. Dans le cadre de la présente invention, on entend par « base forte », une base qui se dissocie totalement au cours de sa réaction avec l'eau.

De manière avantageuse, les bases fortes sont choisies parmi l'hydroxyde de sodium, l'oxyde de potassium, l'hydroxyde de potassium, l'oxyde de césium, l'hydroxyde de césium, l'oxyde de calcium, l'hydroxyde de calcium, l'ion amidure, l'oxyde de baryum et l'hydroxyde de baryum.

La concentration de l'espèce titrante peut être comprise entre 0,01 mol/L et 10 mol/L, de préférence entre 0,5 mol/L et 2 mol/L.

De manière tout à fait avantageuse, la concentration de l'espèce titrante est de 1 mol/L.

Dans un mode de réalisation de l'invention, lorsque le pH de l'échantillon de substrat ou de digestat dont on cherche à estimer la concentration en l'au moins un composé d'intérêt est inférieur ou égal à une 1$^{\text{ière}}$ valeur seuil de pH, le titrage de l'étape b) au cours duquel on collecte des données expérimentales est un titrage basique.

Préférentiellement, ledit titrage basique est réalisé jusqu'à ce que le pH de la solution réactionnelle atteigne une 2$^{\text{ème}}$ valeur seuil de pH.

Dans un mode de réalisation de l'invention, la 2$^{ième}$ valeur seuil de pH est supérieure à la 1$^{ière}$ valeur seuil de pH.

Dans un mode de réalisation de l'invention, lorsque le pH de l'échantillon de substrat ou de digestat dont on cherche à estimer la concentration en l'au moins un composé d'intérêt est supérieur à ladite 1$^{ière}$ valeur seuil de pH, le titrage de l'étape b) au cours duquel on collecte des données expérimentales consiste en :

- un 1$^{ier}$ titrage qui est un titrage acide jusqu'à ce que le pH de la solution réactionnelle atteigne ladite 1$^{ière}$ valeur seuil de pH,
- suivi d'un 2$^{ième}$ titrage qui est un titrage basique.

Le 2$^{ième}$ titrage basique peut être réalisé jusqu'à ce que le pH de la solution réactionnelle atteigne ladite 2$^{ième}$ valeur seuil de pH.

Ladite 1$^{ière}$ valeur seuil de pH peut être comprise entre 0 et 4. De manière préférée, ladite 1$^{ière}$ valeur seuil de pH est 2.

La 2$^{ième}$ valeur seuil de pH peut être comprise entre 10 et 12 si la concentration en azote ammoniacal est estimée au cours du procédé d'estimation selon l'invention.

Si la concentration en azote ammoniacal n'est pas estimée au cours du procédé d'estimation selon l'invention, alors la 2$^{ième}$ valeur seuil de pH peut être comprise entre 6,5 et 12.

De manière préférée, la 2$^{ième}$ valeur seuil de pH est 11,5.

L'au moins une fonction théorique de titrage peut être choisie parmi :

- le volume titrant du titrage en fonction du pH de la solution réactionnelle ;
- la conductivité de la solution réactionnelle en fonction du pH de la solution réactionnelle, ci-après abrégée par « la conductivité en fonction du pH » ;
- la dérivée de la conductivité de la solution réactionnelle en fonction du pH de la solution réactionnelle par le pH de la solution réactionnelle, ci-après abrégée « dérivée de la conductivité par le pH » ;
- la capacité tampon $\beta$, ladite capacité tampon $\beta$ correspondant à la dérivée de la concentration de l'espèce titrante dans la solution réactionnelle en fonction du pH de la solution réactionnelle.

Lorsque le titrage est un titrage basique dont l'espèce titrante est de l'hydroxyde de sodium, une fonction théorique du volume titrant peut être l'équation (I) suivante :

$$V_{titrant} = V_{échantillon} * \frac{(HCO_3^- + 2*CO_3^{2-} + AGV^- - NH_4^+ - Z^+ + Z^- + OH^- - H_3O^+)}{[Na^+]_{titrant} + H_3O^+ - OH^-} \text{ (I)}$$

Lorsque le titrage est un titrage acide dont l'espèce titrante est de l'acide chlorhydrique, une fonction théorique du volume titrant peut être l'équation (II) suivante :

$$V_{titrant} = V_{échantillon} * \frac{(NH_4^+ - Z^- + Z^+ - HCO_3^- - 2*CO_3^{2-} - AGV^- - OH^- + H_3O^+)}{[Cl^-]_{titrant} - H_3O^+ + OH^-}$$

$$\text{(II)}$$

Lorsque le titrage est un titrage basique, une fonction théorique de la capacité tampon $\beta$ peut être l'équation (III) suivante :

$$\beta = \left(\frac{dHCO_3^-}{dpH} + 2 * \frac{dCO_3^{2-}}{dpH} + \frac{dAGV^-}{dpH} - \frac{dNH_4^+}{dpH}\right) * \frac{V_{échantillon}}{V_{échantillon} + V_{titrant}}$$

$$+ \frac{d\frac{V_{échantillon}}{V_{échantillon} + V_{titrant}}}{dpH} * (HCO_3^- + 2 * CO_3^{2-} + AGV^- - NH_4^+ - Z^+$$

$$+ Z^-) + \frac{dOH^-}{dpH} - \frac{dH_3O^+}{dpH}$$

$$\text{(III)}$$

Dans ces équations (I) à (III), NH4$^+$, HCO$_3^-$, CO$_3^{2-}$, AGV$^-$, Z$^+$, Z$^-$, OH$^-$, H$_3$O$^+$ étant respectivement les concentrations de NH4$^+$, HCO$_3^-$, CO$_3^{2-}$, les ions chargés négativement des AGV, les ions dont la charge n'est pas affectée par le pH et qui sont chargés positivement de la solution réactionnelle, les ions dont la charge n'est pas affectée par le pH et qui sont chargés négativement de la solution réactionnelle, OH$^-$ et H$_3$O$^+$ et V$_{échantillon}$ étant le volume de l'échantilon de substrat ou de digestat, V$_{titrant}$ étant le volume de la solution titrante.

Lorsque le titrage est un titrage acide, une fonction théorique de la capacité tampon β peut être l'équation (IV) suivante :

$$\beta = \left(\frac{dNH_4^+}{dpH} - \frac{dHCO_3^-}{dpH} - 2*\frac{dCO_3^{2-}}{dpH} - \frac{dAGV^-}{dpH}\right)*\frac{V_{échantillon}}{V_{échantillon}+V_{titrant}} + \frac{d\frac{V_{échantillon}}{V_{échantillon}+V_{titrant}}}{dpH}*$$
$$(NH_4^+ + Z^+ - Z^- - HCO_3^- - 2*CO_3^{2-} - AGV^-) - \frac{dOH^-}{dpH} + \frac{dH_3O^+}{dpH}$$

$$(IV)$$

avec les mêmes définitions que pour les équations (I) à (III) qui ont été détaillées ci-dessus.

Lorsque le titrage est un titrage acide, suivi d'un titrage basique, une fonction théorique de la capacité tampon β peut être l'équation (V) suivante :

$$\beta = \left(\frac{dHCO_3^-}{dpH} + 2*\frac{dCO_3^{2-}}{dpH} + \frac{dAGV^-}{dpH} - \frac{dNH_4^+}{dpH}\right)$$
$$*\frac{V_{échantillon}^2}{(V_{échantillon}+V_{titrant})*(V_{échantillon}+V_{maxTitrantAcide})}$$
$$+\frac{V_{échantillon}}{(V_{échantillon}+V_{maxTitrantAcide})}*$$
$$\frac{d\frac{V_{échantillon}}{V_{échantillon}+V_{titrant}}}{dpH}*(HCO_3^- + 2*CO_3^{2-} + AGV^- - NH_4^+ - Z^+ + Z^- + Cl^-) + \frac{dOH^-}{dpH} - \frac{dH_3O^+}{dpH}$$

$$(V)$$

avec les mêmes définitions que pour les équations (I) à (III) qui ont été détaillées ci-dessus et en outre pour cette équation (V) : V$_{maxTitrantAcide}$ étant le volume titrant acide total utilisé et Cl$^-$ étant la concentration des ions chlorures (Cl$^-$) qui sont présents dans la solution réactionnelle à la fin du titrage acide du fait de l'ajout de l'acide chlorhydrique au cours de ce titrage acide.

Lorsque le titrage est un titrage acide ou basique, une fonction théorique de la conductivité peut être l'équation (VI) suivante :

$$Conductivité = \lambda_{OH-}*[OH^-] + \lambda_{H3O+}*[H_3O^+] + \left[\lambda_{HCO3-}*[HCO_3^-] + \lambda_{CO32-}*[CO_3^{2-}] +\right.$$
$$\left.\lambda_{Acetate}*[AGV^-] + \lambda_{NH4+}*[NH_4^+] + \lambda_{Cl-}*[Z^-] + \frac{(\lambda_{Na+}+\lambda_{K+})}{2}*[Z^+]\right]*\frac{V_{échantillon}}{V_{échantillon}+V_{titrant}}$$
$$+\lambda_{ions_{titrant}}*[ion]_{titrant}*\frac{V_{titrant}}{V_{échantillon}+V_{titrant}}$$

$$(VI)$$

avec les mêmes définitions que pour les équations (I) à (III) qui ont été détaillées ci-dessus et en outre pour cette équation (VI) : [ion]$_{titrant}$ qui est la molarité de la solution titrante.

La dérivée de cette fonction théorique de la conductivité (VI) peut être la fonction théorique (VII) suivante :

$$\frac{d\text{conductivité}}{dpH} = \frac{d\frac{V_{\text{échantillon}}}{V_{\text{échantillon}} + V_{\text{titrant}}}}{dpH} * (\lambda_{\text{HCO3}-}\text{HCO}_3^- + \lambda_{\text{CO32}-}\text{CO}_3^{2-} + \lambda_{\text{Acetate}}\text{AGV}^- +$$

$$\lambda_{\text{NH4}+}\text{NH}_4^+ + \lambda_{\text{Cl}-}Z^- + \lambda_{\text{Na}+/\text{K}+}Z^+) + \frac{V_{\text{échantillon}}}{V_{\text{échantillon}} + V_{\text{titrant}}} * \left[\lambda_{\text{HCO3}-} * \frac{d\text{HCO}_3^-}{dpH} + \right.$$

$$\lambda_{\text{CO32}-} * \frac{d\text{CO}_3^{2-}}{dpH} + \lambda_{\text{Acetate}} * \frac{d\text{AGV}^-}{dpH} + \lambda_{\text{NH4}+} * \frac{d\text{NH}_4^+}{dpH}\left.\right] + \lambda_{\text{OH}-} * \frac{d\text{OH}^-}{dpH} + \lambda_{\text{H3O}+} * \frac{d\text{H}_3\text{O}^+}{dpH} +$$

$$\lambda_{\text{ions}_{\text{titrant}}} * \beta \qquad \text{(VII)}$$

dans ladite fonction (VII), les ions titrants étant les ions de la solution titrante.

Dans les équations détaillées ci-dessus, les concentrations de NH4$^+$, HCO$_3^-$, CO$_3^{2-}$, des ions chargés négativement des AGV, des ions dont la charge n'est pas affectée par le pH et qui sont chargés positivement de la solution réactionnelle (Z$^+$) et des ions dont la charge n'est pas affectée par le pH et qui sont chargés négativement de la solution réactionnelle (Z$^-$) sont exprimées à volume constant (c'es-à-dire au volume de l'échantillon de substrat ou de digestat). Mais il est tenu compte dans ces équations de l'effet de dilution de ces différentes espèces au cours du titrage du fait que lesdites équations comprennent un facteur de dilution approprié.

De manière avantageuse, les données expérimentales collectées au cours du au moins un titrage de l'échantillon de substrat ou de digestat consistent en au moins un relevé de valeurs expérimentales qui est choisi parmi :

- un relevé du volume titrant de la solution titrante en fonction du pH de la solution réactionnelle à partir duquel on établit une fonction expérimentale f1 : Volume titrant = f1 (pH) ;
- un relevé de la conductivité de la solution réactionnelle en fonction du pH de la solution réactionnelle à partir duquel on établit une fonction expérimentale f2 : conductivité = f2 (pH).

Dans un mode de réalisation de l'invention, les données expérimentales collectées au cours du au moins un titrage de l'échantillon de substrat ou de digestat consistent en au moins un relevé de valeurs expérimentales de la conductivité de la solution réactionnelle en fonction du pH de la solution réactionnelle à partir duquel on établit une fonction expérimentale f2 : conductivité = f2 (pH).

Dans ce mode de réalisation de l'invention, on couple les données expérimentales recueillies par pH-métrie à celles obtenues par conductimétrie. Cela présente l'avantage d'obtenir d'avantage d'informations sur les composés d'intérêt présents dans l'échantillon de substrat ou de digestat dont on cherche à estimer la concentration. Ce couplage donne notamment des informations sur la charge portée par l'espèce titrée. La conductimétrie rend compte du gain ou de la perte de charge de la solution réactionnelle.

Dans un mode de réalisation de l'invention, on supprime parmi les données expérimentales collectées les données expérimentales qui semblent impossibles, ou autrement dit aberrantes, au regard de l'historique des données expérimentales collectées au cours du titrage.

Dans un mode de réalisation de l'invention, on supprime des données expérimentales collectées les données expérimentales qui ont été relevées à la fin du titrage. En effet, il s'agit généralement de données expérimentales qui ne seront pas exploitables pour l'étape c) du procédé d'estimation consistant à identifier une concentration d'au moins un composé d'intérêt pour laquelle la fonction théorique de titrage présente une erreur minimale avec la fonction expérimentale de titrage.

Dans un mode de réalisation de l'invention, les données expérimentales collectées peuvent être soumises à au moins un traitement mathématique et à partir duquel on établit la fonction expérimentale de titrage. Ledit traitement mathématique peut consister en au moins un traitement mathématique choisi parmi :

- un lissage ;
- un calcul de dérivée ;
- un calcul de la capacité tampon $\beta$, ladite capacité tampon $\beta$ correspondant à la dérivée de la concentration de l'espèce titrante dans la solution réactionnelle en fonction du pH de la solution réactionnelle.

Le traitement mathématique de lissage peut consister en une interpolation cubique, de préférence une interpolation cubique avec 15 degrés de liberté.

Dans un mode de réalisation de l'invention, on modélise l'au moins une fonction expérimentale de titrage sous la forme d'une fonction polynomiale.

De manière préférée, l'au moins une fonction expérimentale de titrage est modélisée sous la forme d'une fonction polynomiale de 3$^{\text{ième}}$ degré.

Dans un mode de réalisation de l'invention, l'au moins une fonction expérimentale de titrage modélisée sous la forme d'une fonction polynomiale est soumise à un traitement mathématique de lissage.

Dans un mode de réalisation préféré de l'invention, l'au moins une fonction expérimentale de titrage est modélisée sous la forme d'une fonction polynomiale de 3$^{ième}$ degré, puis soumise à un traitement mathématique de lissage.

Ensuite, un traitement mathématique tel qu'un calcul de dérivée peut être effectué sur cette fonction expérimentale de titrage modélisée et lissée de manière à obtenir une autre fonction expérimentale de titrage.

Le traitement mathématique de lissage sur la fonction expérimentale de titrage est particulièrement avantageux si l'on soumet ensuite cette fonction expérimentale de titrage à un traitement mathématique de calcul de dérivée. En effet, la dérivation de fonctions amplifie le bruit des données. Le traitement de lissage supprime le bruit des données expérimentales (autrement dit filtre les données expérimentales) ; ce qui permet d'obtenir une dérivée de fonction expérimentale présentant un minimum de bruits.

Dans un mode de réalisation de l'invention, le calcul de dérivée consiste en le calcul de dérivée de la fonction expérimentale f2.

Dans un mode de réalisation de l'invention, ledit traitement mathématique consiste en :

- le calcul de dérivée de la fonction expérimentale f2 ; et/ou
- le calcul de ladite capacité tampon $\beta$.

Dans un mode de réalisation de l'invention, l'erreur minimale est obtenue à partir d'une méthode de minimisation qui se base sur la somme des carrés des erreurs entre l'au moins une fonction théorique de titrage et l'au moins une fonction expérimentale de titrage.

Le procédé d'estimation comprend une étape consistant à corriger l'estimation de la concentration en l'au moins un composé d'intérêt présent dans ledit échantillon de substrat ou de digestat de manière à obtenir une estimation corrigée de cette concentration.

La correction de l'estimation consiste à :

- supprimer un écart de pH entre des valeurs de pH relevées au cours dudit titrage lors de la collecte des données expérimentales et des valeurs de pH du modèle de référence d'échantillon de substrat ou de digestat déterminées à partir de la concentration en le au moins un composé d'intérêt qui a été identifiée à l'étape c1),c2) de manière à obtenir des données expérimentales corrigées pour établir à partir de ces données expérimentales corrigées au moins une fonction expérimentale de titrage corrigée, puis
- à partir desdites données expérimentales corrigées, à identifier une 2$^{ième}$ concentration du au moins un composé d'intérêt pour laquelle l'au moins une fonction théorique de titrage du modèle de référence d'échantillon de substrat ou de digestat présente une erreur minimale avec l'au moins une fonction expérimentale de titrage corrigée de manière à estimer la concentration en l'au moins un composé d'intérêt présent dans ledit échantillon de substrat ou de digestat qui est une estimation corrigée de ladite concentration.

Dans un mode de réalisation de l'invention, la suppression de l'écart de pH est réalisée sur les dernières données expérimentales relevées au cours du titrage.

Dans un mode de réalisation de l'invention, la correction de l'estimation consiste à déterminer des valeurs de pKa correspondant aux points d'annulation de la dérivée de l'au moins une fonction théorique de titrage du modèle de référence d'échantillon de substrat ou de digestat qui présentent une erreur minimale avec des valeurs de pKa correspondant aux points d'annulation de la dérivée de l'au moins une fonction expérimentale de titrage corrigée de manière à obtenir une estimation corrigée de la concentration en l'au moins un composé d'intérêt présent dans ledit échantillon de substrat ou de digestat.

Dans ce mode de réalisation de l'invention, l'erreur minimale peut être obtenue à partir d'une méthode de minimisation qui se base sur la somme des carrés des erreurs entre les valeurs de pKa correspondant aux points d'annulation de la dérivée de l'au moins une fonction théorique de titrage du modèle de référence d'échantillon de substrat ou de digestat identifié et les valeurs de pKa correspondants aux points d'annulation de la dérivée de l'au moins une fonction expérimentale de titrage corrigée.

De préférence, les valeurs de pKa sont ajustées entre plus ou moins 1 unité de pH, de préférence entre plus ou moins 0,4 unités de pH.

Le procédé d'estimation comprend une étape au cours de laquelle on vérifie que l'estimation obtenue à l'étape c1) de la concentration en l'au moins un composé d'intérêt présent dans l'échantillon de substrat ou de digestat est compatible avec un modèle de référence d'échantillon de substrat ou de digestat associant la concentration du au moins un composé d'intérêt à au moins une fonction théorique de titrage qui est distincte de l'au moins une fonction théorique de titrage de l'étape a1).

Dans un mode de réalisation de l'invention, le procédé d'estimation comprend une étape au cours de laquelle on vérifie que l'estimation corrigée de la concentration en l'au moins un composé d'intérêt présent dans l'échantillon de substrat ou de digestat est compatible avec un modèle de référence d'échantillon de substrat ou de digestat associant la concentration du au moins un composé d'intérêt à au moins une fonction théorique de titrage qui est distincte de l'au moins une fonction théorique de titrage qui a été utilisée pour la correction de l'estimation de la concentration de l'au moins un composé d'intérêt.

Lorsque le titrage est un titrage acide, alors la fonction théorique de titrage de l'étape a1) est la fonction théorique de la capacité tampon $\beta$ et la fonction expérimentale de titrage de l'étape b1) est la fonction expérimentale de la capacité tampon $\beta$.

Dans ce mode de réalisation de l'invention, le procédé d'estimation peut en outre comprendre une étape au cours de laquelle on vérifie que l'estimation obtenue à l'étape c1) de la concentration en l'au moins un composé d'intérêt présent dans l'échantillon de substrat ou de digestat est compatible avec un modèle de référence d'échantillon de substrat ou de digestat associant ladite concentration du au moins un composé d'intérêt à une fonction théorique de titrage de la conductivité en fonction du pH.

Selon une alternative de ce mode de réalisation de l'invention et lorsque le procédé comprend une étape de correction de l'estimation de la concentration de l'au moins un composé d'intérêt, ledit procédé d'estimation peut en outre comprendre une étape au cours de laquelle on vérifie que l'estimation corrigée de la concentration en l'au moins un composé d'intérêt présent dans l'échantillon de substrat ou de digestat est compatible avec un modèle de référence d'échantillon de substrat ou de digestat associant ladite concentration du au moins un composé d'intérêt à une fonction théorique de titrage de la conductivité en fonction du pH.

Dans ces modes de réalisation de l'invention tels que décrits juste ci-dessus, la capacité tampon $\beta$ ne donnant aucune indication sur la charge portée par les espèces titrées, il est avantageux de vérifier la compatibilité de l'estimation de la concentration de l'au moins un composé d'intérêt avec la conductivité en fonction du pH afin de limiter le risque d'erreur de ladite estimation.

Dans un mode de réalisation de l'invention, le procédé d'estimation selon la revendication 1 met en œuvre un titrage acide et comprend au moins les étapes suivantes :

- a1) On dispose d'un modèle de référence d'échantillon de substrat ou de digestat associant la concentration en carbone inorganique et en AGV de ce modèle de référence d'échantillon de substrat ou de digestat à une fonction théorique de titrage de la capacité tampon $\beta$;

- b1) On établit une fonction expérimentale de titrage de la capacité tampon $\beta$ à partir de données expérimentales collectées au cours dudit titrage acide de l'échantillon de substrat ou de digestat ;

- c1) On identifie au moyen du modèle de référence d'échantillon de substrat ou de digestat une concentration de carbone inorganique et une concentration d'AGV pour lesquelles la fonction théorique de titrage de la capacité tampon $\beta$ de ce modèle de référence d'échantillon de substrat ou de digestat présente une erreur minimale avec la fonction expérimentale de titrage de la capacité tampon $\beta$ de manière à estimer la concentration de carbone inorganique et d'AGV présents dans ledit échantillon de substrat ou de digestat ;

- d1) On vérifie que l'estimation obtenue à l'étape c1) de la concentration de carbone inorganique et d'AGV présents dans l'échantillon de substrat ou de digestat est compatible avec un modèle de référence d'échantillon de substrat ou de digestat associant lesdites concentrations de carbone inorganique et d'AGV à une fonction théorique de la conductivité en fonction du pH ;

- e1) On supprime un écart de pH entre des valeurs de pH mesurées au cours dudit titrage acide et des valeurs de pH du modèle de référence d'échantillon de substrat ou de digestat déterminées à partir des concentrations de carbone inorganique et d'AGV qui ont été identifiées à l'étape c1) de manière à obtenir des données expérimentales corrigées pour établir à partir de ces données expérimentales corrigées une fonction expérimentale de titrage corrigée de la capacité tampon $\beta$ ;

- f1) A partir desdites données expérimentales corrigées, on identifie une $2^{ième}$ concentration de carbone inorganique et une $2^{ième}$ concentration d'AGV pour lesquelles une fonction théorique de titrage de la capacité tampon $\beta$ du modèle de référence d'échantillon de substrat ou de digestat présente une erreur minimale avec la fonction expérimentale de titrage corrigée de la capacité tampon $\beta$ de manière à estimer la concentration corrigée de carbone inorganique et d'AGV présents dans ledit échantillon de substrat ou de digestat ;

- g1) On détermine des valeurs de pKa correspondant aux points d'annulation de la dérivée de la fonction théorique de titrage de la capacité tampon $\beta$ du modèle de référence d'échantillon de substrat ou de digestat qui présentent une erreur minimale avec des pKa correspondants aux points d'annulation de la dérivée de la fonction expérimentale de titrage corrigée de la capacité tampon $\beta$ de manière à obtenir une estimation corrigée de la concentration de carbone inorganique et d'AGV présents dans ledit échantillon de substrat ou de digestat.

Dans tous ces modes de réalisation de l'invention décrits juste ci-dessus dans lesquels le titrage est un titrage acide, l'erreur minimale peut être obtenue à partir d'une méthode de minimisation basée sur la somme des carrés des erreurs telle que mentionnée ci-dessus.

Lorsque le titrage est un titrage basique, alors la fonction théorique de titrage de l'étape a2) est la fonction théorique de la dérivée de la conductivité par le pH et la fonction expérimentale de titrage de l'étape b2) est la fonction expérimentale de la dérivée de la conductivité par le pH.

Dans ce mode de réalisation de l'invention, ledit procédé d'estimation peut comprendre en outre une étape au cours de laquelle on vérifie que l'estimation obtenue à l'étape c2) de la concentration en l'au moins un composé d'intérêt présent dans l'échantillon de substrat ou de digestat est compatible avec un modèle d'échantillon de substrat ou de digestat associant ladite concentration du au moins un composé d'intérêt à une fonction théorique de titrage de la capacité tampon $\beta$.

La dérivée de la conductivité par le pH étant sensible aux charges, deux espèces de charges opposées pourraient masquer leurs signaux. De façon opposée, la capacité tampon $\beta$ ne l'étant pas, leurs effets s'additionneraient. C'est pourquoi, il est avantageux de vérifier la compatibilité de l'estimation de la concentration de l'au moins un composé d'intérêt avec la capacité tampon $\beta$ afin de limiter le risque d'erreur de l'estimation, et ce tout particulièrement lorsque l'échantillon de substrat ou de digestat est complexe (à savoir qu'il présente des composés dont les valeurs de pKa sont proches les unes des autres).

Selon une alternative de ce mode de réalisation de l'invention et lorsque le procédé d'estimation comprend une étape de correction de l'estimation de la concentration en l'au moins un composé d'intérêt, ledit procédé d'estimation peut comprendre en outre une étape au cours de laquelle on vérifie que l'estimation corrigée de la concentration de l'au moins un composé d'intérêt présent dans l'échantillon de substrat ou de digestat est compatible avec un modèle d'échantillon de substrat ou de digestat associant ladite concentration du au moins un composé d'intérêt à une fonction théorique de titrage de la capacité tampon $\beta$.

Dans un mode de réalisation de l'invention, le procédé d'estimation selon la revendication 2 met en œuvre un titrage basique et comprend au moins les étapes suivantes :

- a2) On dispose d'un modèle de référence d'échantillon de substrat ou de digestat associant la concentration d'azote ammoniacal et d'AGV de ce modèle de référence d'échantillon de substrat ou de digestat à une fonction théorique de titrage de la dérivée de la conductivité par le pH ;
- b2) On établit une fonction expérimentale de titrage de la dérivée de la conductivité par le pH à partir de données expérimentales collectées au cours dudit titrage basique de l'échantillon de substrat ou de digestat ;
- c2) On identifie au moyen du modèle de référence d'échantillon de substrat ou de digestat une concentration d'azote ammoniacal et une concentration d'AGV pour lesquelles la fonction théorique de titrage de la dérivée de la conductivité par le pH de ce modèle de référence d'échantillon de substrat ou de digestat présente une erreur minimale avec la fonction expérimentale de titrage de la dérivée de la conductivité par le pH de manière à estimer la concentration d'azote ammoniacal et d'AGV présents dans ledit échantillon de substrat ou de digestat ;
- d2) On supprime un écart de pH entre des valeurs de pH mesurées au cours dudit titrage basique et des valeurs de pH du modèle de référence d'échantillon de substrat ou de digestat déterminées à partir des concentrations d'azote ammoniacal inorganique et d'AGV qui ont été identifiées à l'étape c2) de manière à obtenir des données expérimentales corrigées pour établir une fonction expérimentale de titrage corrigée de la dérivée de la conductivité par le pH ;
- e2) A partir desdites données expérimentales corrigées, on identifie une 2$^{ième}$ concentration d'azote ammoniacal et une 2$^{ième}$ concentration d'AGV pour lesquelles une fonction théorique de titrage de la dérivée de la conductivité par le pH du modèle de référence d'échantillon de substrat ou de digestat présente une erreur minimale avec la fonction expérimentale de titrage corrigée de la dérivée de la conductivité par le pH de manière à estimer la concentration corrigée d'azote ammoniacal et d'AGV présents dans ledit échantillon de substrat ou de digestat ;
- f2) A partir desdites données expérimentales corrigées, on établit une fonction expérimentale de titrage de la capacité tampon $\beta$ ;
- g2) On identifie au moyen du modèle de référence d'échantillon de substrat ou de digestat une 3$^{ième}$ concentration d'azote ammoniacal et une 3$^{ième}$ concentration d'AGV pour lesquelles une fonction théorique de titrage de la capacité tampon $\beta$ du modèle de référence d'échantillon de substrat ou de digestat présente une erreur minimale avec la fonction expérimentale de titrage de la capacité tampon $\beta$ établie à l'étape f2) de manière à estimer la concentration d'azote ammoniacal et d'AGV présents dans ledit échantillon de substrat ou de digestat ;
- h2) On vérifie que l'estimation obtenue à l'étape g2) de la concentration d'azote ammoniacal et d'AGV présents dans l'échantillon de substrat ou de digestat est compatible avec un modèle d'échantillon de substrat ou de digestat associant lesdites concentrations d'azote ammoniacal et d'AGV à une fonction théorique de titrage de la capacité tampon $\beta$ ;
- i2) On détermine des valeurs de pKa correspondant aux points d'annulation de la dérivée de la fonction théorique de titrage de la dérivée de la conductivité par le pH associée au modèle de référence d'échantillon de substrat ou de digestat qui présentent une erreur minimale avec des valeurs de pKa correspondants aux points d'annulation de la dérivée de la fonction expérimentale de titrage corrigée de la dérivée de la conductivité par le pH obtenue à l'étape d2) de manière à obtenir une estimation corrigée de la concentration d'azote ammoniacal et d'AGV présents dans ledit échantillon de substrat ou de digestat ;

- j2) On estime la concentration d'azote ammoniacal et d'AGV de la manière suivante :

    - lorsque l'estimation obtenue à l'étape g2) est compatible, l'estimation de la concentration d'azote ammoniacal et d'AGV correspond à la moyenne des estimations des concentrations d'azote ammoniacal et d'AGV des étapes g2) et i2) ;
    - lorsque l'estimation obtenue à l'étape g2) n'est pas compatible, l'estimation de la concentration d'azote ammoniacal et d'AGV correspond à l'estimation des concentrations d'azote ammoniacal et d'AGV de l'étape i2).

Dans tous ces modes de réalisation de l'invention décrits ci-dessus dans lesquels le titrage est un titrage basique, l'erreur minimale peut être obtenue à partir d'une méthode de minimisation basée sur la somme des carrés des erreurs telle que déjà mentionnée ci-dessus.

Un mode de réalisation du procédé d'estimation selon l'invention peut consister à estimer les concentrations de carbone inorganique, d'azote ammoniacal et d'AGV présents dans l'échantillon de substrat ou de digestat de la manière suivante :

- la concentration de carbone inorganique est estimée selon le procédé d'estimation mettant en œuvre un titrage acide et qui comprend les étapes a1) à g1) telles que décrites ci-dessus ;
- la concentration d'AGV et d'azote ammoniacal est estimée selon le procédé d'estimation mettant en œuvre un titrage acide et qui comprend les étapes a2) à j2) telles que décrites ci-dessus.

Dans le mode de réalisation du procédé d'estimation selon l'invention dans lequel le titrage consiste en un titrage basique, suivi d'un titrage acide, alors on estime les concentrations de carbone inorganique, d'azote ammoniacal et d'AGV de la manière suivante :

- la concentration de carbone inorganique est estimée selon le procédé d'estimation mettant en œuvre un titrage acide et qui comprend les étapes a1) à g1) telles que décrites ci-dessus ;
- la concentration d'AGV et d'azote ammoniacal est estimée selon le procédé d'estimation mettant en œuvre un titrage basique et qui comprend les étapes a2) à j2) telles que décrites ci-dessus.

En effet, au cours d'un titrage acide, l'acidification de l'$HCO_3^-$ en $H_2CO_3$ va avoir de l'influence sur l'estimation de la concentration des AGV présents dans l'échantillon de digestat. C'est pourquoi, pour estimer la concentration des AGV, on prend en considération l'estimation obtenue lorsque le titrage est un titrage basique au cours duquel il ne se produit pas de réaction avec le carbone inorganique. Celui-ci ayant été éliminé à la fin de la titration acide par bullage.

L'invention sera bien comprise à l'aide de la description détaillée qui suit, en référence, au dessin schématique annexé en représentant, à titre d'exemple non limitatif, les étapes du procédé selon l'invention selon deux modes de réalisation, ainsi que les résultats expérimentaux obtenus après la mise en œuvre d'un procédé selon l'invention.

La figure 1 est un schéma synoptique des étapes du procédé selon l'invention selon un 1$^{ier}$ mode de réalisation.
La figure 2 est un schéma synoptique des étapes du procédé selon l'invention selon un 2$^{ième}$ mode de réalisation.
La figure 3 est un graphe détaillant pour 24 échantillons de digestat la concentration en AGV obtenue selon une analyse chimique de référence et selon le procédé d'estimation selon l'invention.
La figure 4 un graphe détaillant pour 24 échantillons de digestat la concentration en azote ammoniacal obtenue selon une analyse chimique de référence et selon le procédé d'estimation selon l'invention.
La figure 5 un graphe détaillant pour 24 échantillons de digestat la concentration en carbone inorganique obtenue selon une analyse chimique de référence et selon le procédé d'estimation selon l'invention.

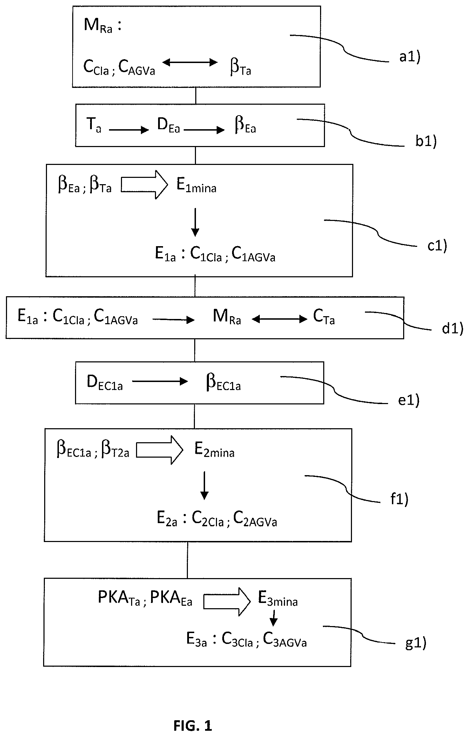

Comme illustré à la figure 1, un 1$^{ier}$ mode de réalisation du procédé selon l'invention consiste en un procédé d'estimation de la concentration du carbone inorganique et des AGV présents dans un échantillon d'un substrat ou d'un digestat qui comprend les étapes suivantes :

- une étape a1) consistant à disposer d'un modèle de référence d'échantillon de substrat ou de digestat $M_{Ra}$ associant la concentration de carbone inorganique et d'AGV ($C_{Cla}$ et $C_{AGVa}$) de ce modèle de référence d'échantillon de substrat ou de digestat $M_{Ra}$ à une fonction théorique de titrage de la capacité tampon $\beta_{Ta}$ ;
- une étape b1) consistant à établir une fonction expérimentale de titrage de la capacité tampon $\beta_{Ea}$ à partir de données expérimentales $D_{Ea}$ collectées au cours d'un titrage acide $T_a$ de l'échantillon de substrat ou de digestat ;

- une étape c1) consistant à identifier au moyen du modèle de référence d'échantillon de substrat ou de digestat $M_{Ra}$ une concentration de carbone inorganique et une concentration d'AGV ($C_{1Cla}$, $C_{1AGVa}$) pour lesquelles la fonction théorique de titrage de la capacité tampon $\beta_{Ta}$ de ce modèle de référence d'échantillon de substrat ou de digestat $M_{Ra}$ présente une erreur minimale $E_{1mina}$ avec la fonction expérimentale de titrage de la capacité tampon $\beta_{Ea}$ de manière à estimer $E_{1a}$ la concentration de carbone inorganique et d'AGV ($C_{1Cla}$, $C_{1AGVa}$) présents dans ledit échantillon de substrat ou de digestat ;
- une étape d1) consistant à vérifier que l'estimation $E_{1a}$ de l'étape c1) de la concentration de carbone inorganique et d'AGV ($C_{1Cla}$, $C_{1AGVa}$) présents dans l'échantillon de substrat ou de digestat est compatible avec un modèle de référence d'échantillon de substrat ou de digestat $M_{Ra}$ associant lesdites concentrations de carbone inorganique et d'AGV ($C_{1Cla}$, $C_{1AGV}a$) à une fonction théorique de la conductivité en fonction du pH ($C_{Ta}$) ;
- une étape e1) consistant à supprimer un écart de pH entre des valeurs de pH mesurées au cours dudit titrage acide $T_a$ et des valeurs de pH du modèle de référence d'échantillon de substrat ou de digestat $M_{Ra}$ déterminées à partir des concentrations de carbone inorganique et d'AGV ($C_{1Cla}$, $C_{1AGVa}$) qui ont été identifiées à l'étape c1) de manière à obtenir des données expérimentales corrigées $D_{EC1a}$ pour établir à partir de ces données expérimentales corrigées $D_{EC1a}$ une fonction expérimentale de titrage corrigée de la capacité tampon $\beta_{EC1a}$ ;
- une étape f1) au cours de laquelle à partir desdites données expérimentales corrigées $D_{EC1a}$, on identifie une $2^{ième}$ concentration de carbone inorganique et une $2^{ième}$ concentration d'AGV ($C_{2Cla}$, $C_{2AGVa}$) pour lesquelles une fonction théorique de titrage de la capacité tampon $\beta$ ($\beta_{T2a,}$) du modèle de référence d'échantillon de substrat ou de digestat $M_{Ra}$ présente une erreur minimale $E_{2mina}$ avec la fonction expérimentale de titrage corrigée de la capacité tampon $\beta$ ($b_{EC1a}$) de manière à estimer $E_{2a}$ la concentration de carbone inorganique et d'AGV ($C_{2Cla}$, $C_{2AGVa}$) présents dans ledit échantillon de substrat ou de digestat ;
- une étape g1) au cours de laquelle on détermine des valeurs de pKa ($PKA_{Ta}$) correspondant aux points d'annulation de la dérivée de la fonction théorique de titrage de la capacité tampon $\beta$ du modèle de référence d'échantillon de substrat ou de digestat $M_{Ra}$ qui présentent une erreur minimale $E_{3mina}$ avec des valeurs de pKa ($PKA_{Ea}$) correspondants aux points d'annulation de la dérivée de la fonction expérimentale de titrage corrigée de la capacité tampon $\beta$ ($\beta_{EC1a}$) de manière à obtenir une estimation corrigée $E_{3a}$ de la concentration de carbone inorganique et d'AGV présents dans ledit échantillon de substrat ou de digestat ($C_{3Cla}$, $C_{3AGVa}$).

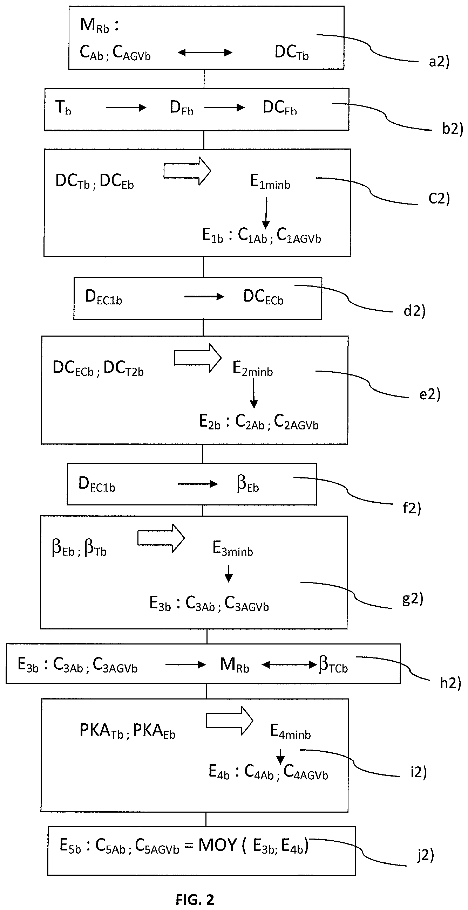

Comme illustré à la figure 2, un $2^{ième}$ mode de réalisation du procédé selon l'invention consiste en un procédé d'estimation de la concentration de l'azote ammoniacal et des AGV présents dans un échantillon d'un substrat ou d'un digestat qui comprend les étapes suivantes :

- une étape a2) consitant à disposer d'un modèle de référence d'échantillon de substrat ou de digestat $M_{Rb}$ associant la concentration d'azote ammoniacal et d'AGV ($C_{Ab}$ et $C_{AGVb}$) de ce modèle de référence d'échantillon de substrat ou de digestat $M_{Rb}$ à une fonction théorique de titrage de la dérivée de la conductivité par le pH ($DC_{Tb}$) ;
- une étape b2) consistant à établir une fonction expérimentale de titrage de la dérivée de la conductivité par le pH ($DC_{Eb}$) à partir de données expérimentales collectées $D_{Eb}$ au cours d'un titrage basique $T_b$ de l'échantillon de substrat ou de digestat ;
- une étape c2) consistant à identifier au moyen du modèle de référence d'échantillon de substrat ou de digestat $M_{Rb}$ une concentration d'azote ammoniacal et d'AGV ($C_{1Ab}$, $C_{1AGVb}$) pour lesquelles la fonction théorique de titrage de la dérivée de la conductivité par le pH ($DC_{Tb}$) de ce modèle de référence d'échantillon de substrat ou de digestat $M_{Rb}$ présente une erreur minimale $E_{1minb}$ avec la fonction expérimentale de titrage de la dérivée de la conductivité par le pH ($DC_{Eb}$) de manière à estimer $E_{1b}$ la concentration d'azote ammoniacal et d'AGV ($C_{1Ab}$, $C_{1AGVb}$) présents dans ledit échantillon de substrat ou de digestat ;
- une étape d2) au cours de laquelle on supprime un écart de pH entre des valeurs de pH mesurées au cours dudit titrage basique $T_b$ et des valeurs de pH du modèle de référence d'échantillon de substrat ou de digestat $M_{Rb}$ déterminées à partir des concentrations d'azote ammoniacal et d'AGV ($C_{1Ab}$, $C_{1AGVb}$) de manière à obtenir des données expérimentales corrigées $D_{EC1b}$ pour établir une fonction expérimentale de titrage corrigée de la dérivée de la conductivité par le pH ($DC_{ECb}$) ;
- une étape e2) au cours de laquelle, à partir desdites données expérimentales corrigées $D_{EC1b}$, on identifie une $2^{ième}$ concentration d'azote ammoniacal et une $2^{ième}$ concentration d'AGV ($C_{2Ab}$, $C_{2AGVb}$) pour lesquelles une fonction théorique de titrage de la dérivée de la conductivité par le pH ($DC_{T2b}$) de ce modèle de référence d'échantillon de substrat ou de digestat $M_{Rb}$ présente une erreur minimale $E_{2minb}$ avec la fonction expérimentale de titrage corrigée de la dérivée de la conductivité par le pH ($DC_{ECb}$) de manière à estimer $E_{2b}$ la concentration corrigée d'azote ammoniacal et d'AGV ($C_{2Ab}$, $C_{2AGVb}$) présents dans ledit échantillon de substrat ou de digestat ;
- une étape f2) au cours de laquelle à partir desdites données expérimentales corrigées $D_{EC1b}$, on établit une fonction expérimentale de titrage de la capacité tampon $\beta$ ($\beta_{Eb}$) ;
- Une étape g2) au cours de laquelle on identifie au moyen du modèle de référence d'échantillon de substrat ou de

digestat $M_{Rb}$ une 3$^{ième}$ concentration d'azote ammoniacal et une 3$^{ième}$ concentration d'AGV ($C_{3Ab}$, $C_{3AGVb}$) pour lesquelles une fonction théorique de titrage de capacité tampon $\beta$ ($\beta_{Tb}$) du modèle de référence d'échantillon de substrat ou de digestat $M_{Rb}$ présente une erreur minimale $E_{3minb}$ avec la fonction expérimentale de titrage de la capacité tampon $\beta$ ($\beta_{Eb}$) établie à l'étape f2) de manière à estimer $E_{3b}$ la concentration corrigée d'azote ammoniacal et d'AGV présents dans ledit échantillon de substrat ou de digestat ($C_{3Ab}$, $C_{3AGVb}$) ;

- une étape h2) au cours de laquelle on vérifie que l'estimation corrigée $E_{3b}$ obtenue à l'étape g2) de la concentration d'azote ammoniacal et d'AGV ($C_{3Ab}$, $C_{3AGVb}$) présents dans l'échantillon de substrat ou de digestat est compatible avec le modèle de référence d'échantillon de substrat ou de digestat $M_{Rb}$ associant lesdites concentrations d'azote ammoniacal et d'AGV ($C_{3Ab}$, $C_{3AGVb}$) à une fonction théorique de titrage de la capacité tampon $\beta$ ($\beta_{TCb}$) ;
- une étape i2) au cours de laquelle on détermine des valeurs de pKa ($PKA_{Tb}$) correspondant aux points d'annulation de la dérivée de la fonction théorique de titrage de la dérivée de la conductivité par le pH ($DC_{T2b}$) associée au modèle de référence d'échantillon de substrat ou de digestat $M_{Rb}$ qui présentent une erreur minimale $E_{4minb}$ avec des valeurs de pKa ($PKA_{Eb}$) correspondants aux points d'annulation de la dérivée de la fonction expérimentale de titrage corrigée de la dérivée de la conductivité par le pH ($DC_{ECb}$) obtenue à l'étape d2) de manière à obtenir une estimation corrigée $E_{4b}$ de la concentration d'azote ammoniacal et d'AGV ($C_{4Ab}$, $C_{4AGVb}$) présents dans ledit échantillon de substrat ou de digestat;
- une étape j2) au cours de laquelle on estime $E_{5b}$ la concentration d'azote ammoniacal et d'AGV ($C_{5Ab}$, $C_{5AGVb}$) de la manière suivante :

  - lorsque l'estimation corrigée de l'étape g2) est compatible, l'estimation de la concentration d'azote ammoniacal et d'AGV correspond à la moyenne « MOY » des estimations des concentrations d'azote ammoniacal et d'AGV des étapes g2) et i2) ;
  - lorsque l'estimation corrigée de l'étape g2) n'est pas compatible, l'estimation de la concentration d'azote ammoniacal et d'AGV correspond à l'estimation des concentrations d'azote ammoniacal et d'AGV de l'étape i2).

PARTIE EXPERIMENTALE :

**[0101]** Le procédé d'estimation selon l'invention a été testé sur 24 différents échantillons de digestat qui étaient représentatifs de digestats de méthaniseur.

**[0102]** Les 24 échantillons de digestat étaient des digestats de vinasse, d'exploitation agricole, d'unités collectives, d'effluents industriels et de stations d'épuration des eaux usées (ci-après abrégé « STEP »).

**[0103]** Plus précisément, les échantillons 1) à 24) étaient les suivants :

- échantillon n°1) provenant d'une unité traitant des déchets territoriaux de type fumier, agro-déchets, boues de STEP ;
- échantillon n°2) provenant d'unité traitant des déchets territoriaux de type fumier, agro-déchets, boues de STEP ;
- échantillon n°3) provenant d'une unité traitant des déchets agricole type fumier et céréales ;
- échantillon n°4) provenant d'une unité traitant des déchets agricole type fumier et céréales ;
- échantillon n°5) provenant d'une unité traitant des effluents de STEP ;
- échantillon n°6) provenant d'une unité traitant des effluents de STEP ;
- échantillon n°7) provenant d'une unité traitant de la vinasse ;
- échantillon n°8) provenant d'une unité traitant de la vinasse ;
- échantillon n°9) provenant d'une unité traitant de la vinasse ;
- échantillon n°10) provenant d'une unité traitant de la vinasse ;
- échantillon n°11) provenant d'une unité traitant des déchets agricole type fumier et céréales,
- échantillon n°12) provenant d'une unité traitant de la vinasse ;
- échantillon n°13) provenant d'une unité traitant des déchets territoriaux de type fumier, agro-déchets, boues de STEP ;
- échantillon n°14) provenant d'une unité traitant des déchets territoriaux de type fumier, agro-déchets, boues de STEP ;
- échantillon n°15) provenant d'une unité traitant des effluents industriels ;
- échantillon n°16) provenant d'une unité traitant des effluents industriels ;
- échantillon n°17) provenant d'une unité traitant des effluents de papeterie ;
- échantillon n°18) provenant d'une unité traitant des effluents de papeterie ;
- échantillon n°19) provenant d'une unité traitant des déchets territoriaux de type fumier, agro-déchets, boues de STEP ;
- échantillon n°20) provenant d'une unité traitant des déchets territoriaux de type fumier, agro-déchets, boues de STEP ;
- échantillon n°21) provenant d'une unité traitant des déchets agricole type fumier et céréales ;

- échantillon n°22) provenant d'une unité traitant des effluents de ferme et fromagerie ;
- échantillon n°23) provenant d'une unité traitant des effluents de ferme et fromagerie ;
- échantillon n°24) provenant d'une unité traitant des déchets territoriaux de type fumier, agro-déchets, boues de STEP.

On a estimé les concentrations d'AGV, d'azote ammoniacal et de carbone inorganique présents dans ces 24 échantillons de digestat, et ce :

a) à partir du procédé d'estimation selon l'invention ;
b) à partir d'une analyse chimique, dite de référence, qui a permis de valider le procédé d'estimation selon l'invention. L'analyse chimique mise en œuvre pour chacun de ces trois composés inhibiteurs est décrite ci-après.

On a procédé de la manière suivante pour les 24 échantillons de digestat :

- Lorsque le digestat était un digestat liquide, on a prélevé 2,2L de ce digestat liquide. On a conservé 200 mL de ce digestat liquide pour l'analyse chimique détaillée ci-après et on a mis en œuvre le procédé d'estimation selon l'invention sur les 2 L.
- Lorsque le digestat était un digestat solide (à savoir que le pourcentage massique de sa matière sèche était supérieur à 10%), on a préparé une dilution au 1/10^{ième} de ce digestat solide dans de l'eau distillée. Puis, on a réalisé une filtration grossière de 2,2L de la solution ainsi obtenue à l'issue de l'aide, et ce à l'aide d'une passoire. On a conservé 200 mL de ce digestat liquide ainsi obtenu pour l'analyse chimique détaillée ci-après et on mis en œuvre le procédé d'estimation selon l'invention sur les 2 L.

L'analyse chimique de référence pour l'estimation de la concentration des AGV présents dans les échantillons de digestat a été réalisée par chromatographie en phase gazeuse.

Pour ce faire, on a utilisé un appareillage de chromatographie en phase gazeuse de la société PerkinElmer et connu sous la dénomination « Clarus 580 », La méthode employée était la méthode LBE "Methode-AGV-17min".

L'analyse chimique de référence pour l'estimation de la concentration du carbone inorganique présent dans les échantillons de digestat a été réalisée avec un COT-mètre de la société SHIMADZU et connu sous la dénomination « On-line TOC-V$_{CSH}$. La méthode était la méthode « liquid Inorganic Carbon ».

L'analyse chimique de référence pour l'estimation de la concentration de l'azote ammoniacal présent dans les échantillons de digestat a été réalisée avec un appareillage de la société Buchi connu sous la dénomination « AutoKjeldahl Unit K-370 ». La méthode utilisée était la méthode Kjeldhal, sans hydrolyse acide.

Le procédé d'estimation selon l'invention a été mis en œuvre sur les 24 échantillons de digestat de la manière suivante :

On a utilisé un bioréacteur pour effectuer le titrage suivi par pH-métrie et par conductimétrie.

Le bioréacteur était équipé :

- de pompes d'alimentation servaient à l'ajout d'acide chlorhydrique et de soude à des concentrations de 1 mol/L ;
- d'une sonde de pH/Redox/température ;
- d'une sonde température/conductivité.

La sonde de pH était une sonde Mettler Toledo InPro 3253i/SG qui est conçue pour fonctionner avec précision sur une gamme de pH allant de 0 à 12 et de température comprise entre 0 et 100°C.

La sonde de conductivité utilisée était une sonde Mettler Toledo InPro 7100i/12/120. Sa constante de cellule nominale était de 0,31 cm$^{-1}$. Elle possède une précision supérieure à 5% sur une gamme de mesure de 0,02 à 500 ms/cm et de -20°C à 150 °C.

Une légère fuite était tolérée dans une fiole contenant de la soude afin d'éviter la surpression et de filtrer les gaz de sortie.

Afin de s'affranchir des problèmes de dégazage, le bioréacteur était semiétanche.

L'ajout de la solution titrante a été réalisé de manière continue au moyen d'une pompe préalablement calibrée.

L'acquisition des données en ligne a été effectuée par ordinateur, via un logiciel connu sous la dénomination « ODIN 5.2 ».

Le titrage a été réalisé en effectuant les étapes suivantes :

On a tout d'abord effectué un titrage acide sur les 2 L d'échantillon de substrat ou de digestat avec une solution d'acide chlorhydrique à une concentration de 1 mol/L, à un débit de 4 mL/minute et sous une agitation mécanique de 530 tours/minute, jusqu'à ce que le pH de la solution réactionnelle atteigne une valeur de 2.

Ensuite, on a effectué un bullage (0,7L/minute équivalent eau) en maintenant une agitation de 530 tours/minute pendant une durée de 20 minutes.

Enfin, on a effectué un titrage basique avec une solution de soude de concentration 1 mol/L à un débit de 4 mL/minute, et sous une agitation mécanique de 530 tours/minute, jusqu'à ce que le pH de la solution réactionnelle atteigne une valeur de 11,5.

Ce qui a permis d'enregistrer toutes les 15 secondes les valeurs de pH, conductivité, potentiel redox, pression, température, agitation, état de fonctionnement des pompes d'acide chlorhydrique et de soude, ainsi que leurs temps de fonctionnement associés.

La concentration de carbone inorganique a été estimée selon le procédé d'estimation tel que détaillé ci-dessus lorsque le titrage est un titrage acide.

Les concentrations d'AGV et d'azote ammoniacal ont estimées selon le procédé d'estimation tel que détaillé ci-dessus lorsque le titrage est un titrage basique.

Dans le tableau 1 ci-dessous sont détaillées les concentrations (exprimées en mol/L) de :

- AGV estimé selon le procédé selon l'invention (colonne intitulée « [AGV]inv ») ;
- AGV estimé selon le procédé l'analyse chimique de référence (colonne intitulée « [AGV]ref ») ;
- carbone inorganique estimées selon le procédé selon l'invention (colonne intitulée « [CI]inv ») ;
- carbone inorganique estimée selon l'analyse chimique de référence (colonne intitulée « [CI]ref ») ;
- azote ammoniacal estimée selon le procédé selon l'invention (colonne intitullée « [A]inv ») ;
- azote ammoniacal estimée selon le procédé l'analyse chimique de référence (colonne intitulée « [A]ref »).

*Tableau 1 détaillant les concentrations d'AGV, de carbone inorganique et d'azote ammoniacal des 24 échantillons de digestats estimées selon le procédé de l'invention et selon une analyse chimique de référence*

| N° échantillon | [AGV]inv | [AGV]ref | [CI] inv | [CI] ref | [A]inv | [A]ref |
|---|---|---|---|---|---|---|
| 1 | 0,0030 | 0,0030 | 0,1230 | 0,0978 | 0,0900 | 0,0900 |
| 2 | 0,0197 | 0,0197 | 0,1239 | 0,0978 | 0,0900 | 0,0900 |
| 3 | 0,0086 | 0,0050 | 0,0940 | 0,1106 | 0,0365 | 0,0311 |
| 4 | 0,0335 | 0,0385 | 0,1104 | 0,1106 | 0,0590 | 0,0590 |
| 5 | 0,0017 | 0,0000 | 0,0873 | 0,1158 | 0,0638 | 0,0611 |
| 6 | 0,0026 | 0,0000 | 0,0855 | 0,1158 | 0,0497 | 0,0611 |
| 7 | 0,0116 | 0,0130 | 0,0323 | 0,0289 | 0,0000 | 0,0000 |
| 8 | 0,0121 | 0,0130 | 0,0302 | 0,0289 | 0,0000 | 0,0000 |
| 9 | 0,0129 | 0,0130 | 0,0304 | 0,0269 | 0,1396 | 0,1638 |
| 10 | 0,0073 | 0,0065 | 0,0134 | 0,0130 | 0,0733 | 0,0778 |
| 11 | 0,0000 | 0,0009 | 0,0446 | 0,0655 | 0,0395 | 0,0328 |
| 12 | 0,0110 | 0,0130 | 0,0294 | 0,0269 | 0,1518 | 0,1617 |
| 13 | 0,0272 | 0,0307 | 0,0988 | 0,1054 | 0,0452 | 0,0522 |
| 14 | 0,0125 | 0,0110 | 0,1944 | 0,2108 | 0,0833 | 0,1044 |
| 15 | 0,0000 | 0,0007 | 0,1338 | 0,1317 | 0,0411 | 0,0341 |
| 16 | 0,0000 | 0,0007 | 0,1260 | 0,1317 | 0,0289 | 0,0341 |
| 17 | 0,0038 | 0,0000 | 0,0320 | 0,0375 | 0,0070 | 0,0000 |
| 18 | 0,0065 | 0,0000 | 0,0378 | 0,0375 | 0,1012 | 0,0755 |
| 19 | 0,0000 | 0,0040 | 0,1023 | 0,1148 | 0,0643 | 0,0603 |
| 20 | 0,0258 | 0,0293 | 0,1050 | 0,1148 | 0,0682 | 0,0603 |
| 21 | 0,0180 | 0,0246 | 0,0540 | 0,0540 | 0,0410 | 0,0363 |
| 22 | 0,0218 | 0,0251 | 0,0167 | 0,0200 | 0,0013 | 0,0117 |
| 23 | 0,0002 | 0,0020 | 0,0061 | 0,0067 | 0,0100 | 0,0039 |

(suite)

| N° échantillon | [AGV]inv | [AGV]ref | [CI] inv | [CI] ref | [A]inv | [A]ref |
|---|---|---|---|---|---|---|
| 24 | 0,0000 | 0,0020 | 0,2278 | 0,2154 | 0,1476 | 0,1350 |

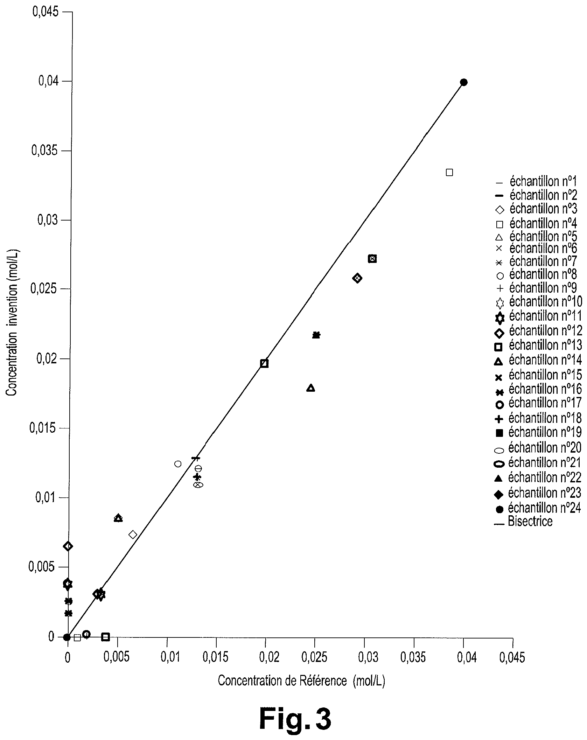

La figure 3 est un graphe détaillant pour les 24 échantillons de digestat en abscisse la concentration d'AGV obtenue selon une analyse chimique de référence et en ordonnée selon le procédé d'estimation selon l'invention.

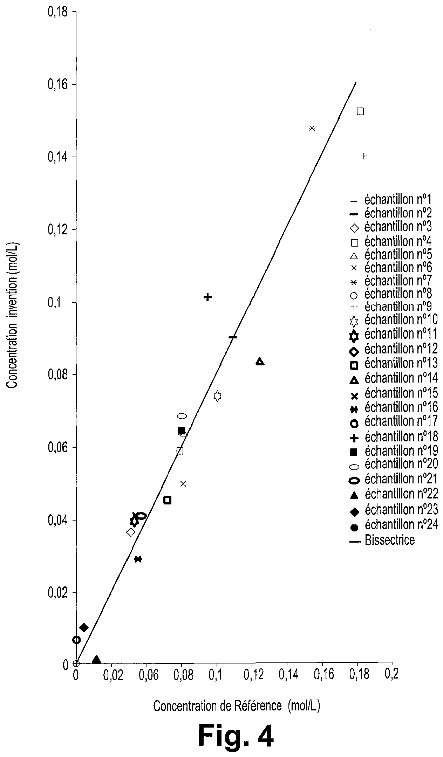

La figure 4 un graphe détaillant pour les 24 échantillons de digestat en abscisse la concentration d'azote ammoniacal obtenue selon une analyse chimique de référence et en ordonnée selon le procédé d'estimation selon l'invention.

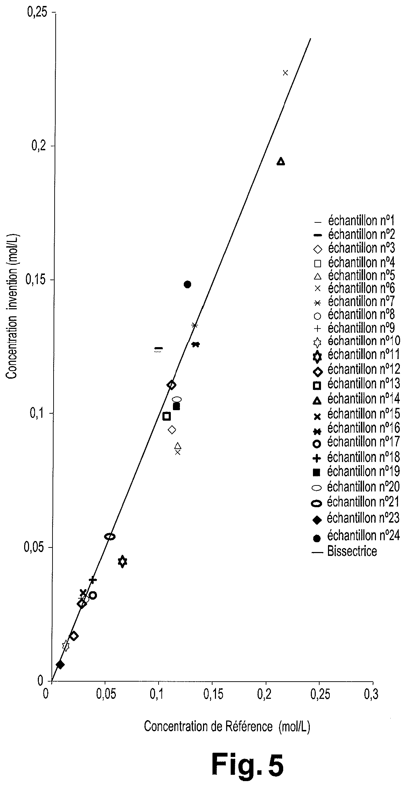

La figure 5 un graphe détaillant pour les 24 échantillons de digestat en abscisse la concentration de carbone inorganique obtenue selon une analyse chimique de référence et en ordonnée selon le procédé d'estimation selon l'invention.

Sur les graphes des figures 3 à 5, la bissectrice a été tracée.

Au vu des graphes des figures 3 à 5, on relève que les estimations des concentrations d'AGV, d'azote ammoniacal et de carbone inorganique fournies par le procédé selon l'invention sont très proches des estimations des concentrations obtenues avec les analyses chimiques de référence. En effet, les points des 24 échantillons de digestat sont très proches sur chacun des trois graphes de la bissectrice.

Ainsi, le procédé d'estimation selon l'invention procure des résultats très satisfaisants quant à l'estimation des composés d'intérêt présents dans des digestats de méthaniseurs tels que les AGV, l'azote ammoniacal et le carbone inorganique, et ce quelle que soit la complexité de digestats.

## Revendications

1. Procédé d'estimation de la concentration de composés d'intérêt choisis parmi le carbone inorganique et les acides gras volatils (abrégé « AGV ») présents dans un échantillon d'un substrat ou d'un digestat d'un méthaniseur, **caractérisé en ce que** ledit procédé d'estimation met en œuvre un titrage acide ($T_a$) et qu'il comprend au moins les étapes suivantes :

   a1) On dispose d'un modèle de référence d'échantillon de substrat ou de digestat ($M_{Ra}$) associant la concentration en carbone inorganique et en AGV ($C_{Cla}$, $C_{AGVa}$) de ce modèle de référence d'échantillon de substrat ou de digestat ($M_{Ra}$) à une fonction théorique de titrage de la capacité tampon β ($β_{Ta}$) ;

   b1) On établit une fonction expérimentale de titrage de la capacité tampon β ($β_{Ea}$) à partir de données expérimentales ($D_{Ea}$) collectées au cours dudit titrage acide ($T_a$) de l'échantillon de substrat ou de digestat ;

   c1) On identifie au moyen du modèle de référence d'échantillon de substrat ou de digestat une concentration de carbone inorganique et une concentration d'AGV ($C_{1Cla}$, $C_{1AGVa}$) pour lesquelles la fonction théorique de titrage de la capacité tampon β ($β_{Ta}$) de ce modèle de référence d'échantillon de substrat ou de digestat ($M_{Ra}$) présente une erreur minimale ($E_{1mina}$) avec la fonction expérimentale de titrage de la capacité tampon β ($β_{Ea}$) de manière à estimer ($E_{1a}$) la concentration de carbone inorganique et d'AGV présents dans ledit échantillon de substrat ou de digestat ($C_{1Cla}$, $C_{1AGVa}$) ;

   d1) On vérifie que l'estimation ($E_{1a}$) obtenue à l'étape c1) de la concentration de carbone inorganique et d'AGV ($C_{1Cla}$, $C_{1AGVa}$) présents dans l'échantillon de substrat ou de digestat est compatible avec un modèle de référence d'échantillon de substrat ou de digestat ($M_{Ra}$) associant lesdites concentrations de carbone inorganique et d'AGV ($C_{1Cla}$, $C_{1AGVa}$) à une fonction théorique de la conductivité en fonction du pH ($C_{Ta}$) ;

   e1) On supprime un écart de pH entre des valeurs de pH mesurées au cours dudit titrage acide ($T_a$) et des valeurs de pH du modèle de référence d'échantillon de substrat ou de digestat ($M_{Ra}$) déterminées à partir des concentrations de carbone inorganique et d'AGV ($C_{1Cla}$, $C_{1AGVa}$) qui ont été identifiées à l'étape c1) de manière à obtenir des données expérimentales corrigées ($D_{EC1a}$) pour établir à partir de ces données expérimentales corrigées ($D_{EC1a}$) une fonction expérimentale de titrage corrigée de la capacité tampon β ($β_{EC1a}$);

   f1) A partir desdites données expérimentales corrigées ($D_{EC1a}$), on identifie une 2$^{ième}$ concentration de carbone inorganique et une 2$^{ième}$ concentration d'AGV ($C_{2Cla}$, $C_{2AGVa}$) pour lesquelles une fonction théorique de titrage de la capacité tampon β ($β_{T2a}$) du modèle de référence d'échantillon de substrat ou de digestat ($M_{Ra}$) présente une erreur minimale ($E_{2mina}$) avec la fonction expérimentale de titrage corrigée de la capacité tampon β ($β_{EC1a}$) de manière à estimer $E_{2a}$ la concentration corrigée de carbone inorganique et d'AGV ($C_{2Cla}$, $C_{2AGVa}$) présents dans ledit échantillon de substrat ou de digestat ;

   g1) On détermine des valeurs de pKa ($PKA_{Ta}$) correspondant aux points d'annulation de la dérivée de la fonction théorique de titrage de la capacité tampon $\beta$ ($\beta_{T2a}$) du modèle de référence d'échantillon de substrat ou de digestat ($M_{Ra}$) qui présentent une erreur minimale avec des pKa ($PKA_{Ea}$) correspondants aux points d'annulation de la dérivée de la fonction expérimentale de titrage corrigée de la capacité tampon $\beta$ ($P_{EC1a}$) de manière à obtenir une estimation corrigée ($E_{3a}$) de la concentration de carbone inorganique et d'AGV présents dans ledit échantillon de substrat ou de digestat ($C_{3Cla}$, $C_{3AGVa}$).

**2.** Procédé d'estimation de la concentration de composés d'intérêts choisis parmi l'azote ammoniacal et les AGV présents dans un échantillon d'un substrat ou d'un digestat d'un méthaniseur, **caractérisé en ce que** ledit procédé d'estimation met en œuvre un titrage basique ($T_b$) et comprend au moins les étapes suivantes :

- a2) On dispose d'un modèle de référence d'échantillon de substrat ou de digestat ($M_{Rb}$) associant la concentration d'azote ammoniacal et d'AGV ($C_{Ab}$ et $C_{AGVb}$) de ce modèle de référence d'échantillon de substrat ou de digestat ($M_{Rb}$) à une fonction théorique de titrage de la dérivée de la conductivité par le pH ($DC_{Tb}$) ;

- b2) On établit une fonction expérimentale de titrage de la dérivée de la conductivité par le pH ($DC_{Eb}$) à partir de données expérimentales ($D_{Eb}$) collectées au cours dudit titrage basique ($T_b$) de l'échantillon de substrat ou de digestat ;

- c2) On identifie au moyen du modèle de référence d'échantillon de substrat ou de digestat ($M_{Rb}$) une concentration d'azote ammoniacal et une concentration d'AGV ($C_{1Ab}$, $C_{1AGVb}$) pour lesquelles la fonction théorique de titrage de la dérivée de la conductivité par le pH ($DC_{Tb}$) de ce modèle de référence d'échantillon de substrat ou de digestat ($M_{Rb}$) présente une erreur minimale ($E_{1minb}$) avec la fonction expérimentale de titrage de la dérivée de la conductivité par le pH ($DC_{Eb}$) de manière à estimer ($E_{1b}$) la concentration d'azote ammoniacal et d'AGV présents dans ledit échantillon de substrat ou de digestat ($C_{1Ab}$, $C_{1AGVb}$) ;

- d2) On supprime un écart de pH entre des valeurs de pH mesurées au cours dudit titrage basique ($T_b$) et des valeurs de pH du modèle de référence d'échantillon de substrat ou de digestat ($M_{Rb}$) déterminées à partir des concentrations d'azote ammoniacal inorganique et d'AGV ($C_{1Ab}$, $C_{1AGVb}$) qui ont été identifiées à l'étape c2) de manière à obtenir des données expérimentales corrigées ($D_{EC1b}$) pour établir une fonction expérimentale de titrage corrigée de la dérivée de la conductivité par le pH ($DC_{ECb}$) ;

- e2) A partir desdites données expérimentales corrigées ($DC_{ECb}$), on identifie une 2$^{ième}$ concentration d'azote ammoniacal et une 2$^{ième}$ concentration d'AGV ($C_{2Ab}$, $C_{2AGVb}$) pour lesquelles une fonction théorique de titrage de la dérivée de la conductivité par le pH ($DC_{T2b}$) du modèle de référence d'échantillon de substrat ou de digestat ($M_{Rb}$) présente une erreur minimale ($E_{2minb}$) avec la fonction expérimentale de titrage corrigée de la dérivée de la conductivité par le pH ($DC_{ECb}$) de manière à estimer ($E_{2b}$) la concentration corrigée d'azote ammoniacal et d'AGV présents ($C_{2Ab}$, $C_{2AGVb}$) dans ledit échantillon de substrat ou de digestat;

- f2) A partir desdites données expérimentales corrigées ($D_{EC1b}$), on établit une fonction expérimentale de titrage de la capacité tampon $\beta$ ($\beta_{Eb}$) ;

- g2) On identifie au moyen du modèle de référence d'échantillon de substrat ou de digestat ($M_{Rb}$) une 3$^{ième}$ concentration d'azote ammoniacal et une 3$^{ième}$ concentration d'AGV ($C_{3Ab}$, $C_{3AGVb}$) pour lesquelles une fonction théorique de titrage de la capacité tampon $\beta$ ($\beta_{Tb}$) du modèle de référence d'échantillon de substrat ou de digestat ($M_{Rb}$) présente une erreur minimale ($E_{3minb}$) avec la fonction expérimentale de titrage de la capacité tampon $\beta$ ($\beta_{Eb}$) établie à l'étape f2) de manière à estimer la concentration d'azote ammoniacal et d'AGV présents ($C_{3Ab}$, $C_{3AGVb}$) dans ledit échantillon de substrat ou de digestat ;

- h2) On vérifie que l'estimation ($E_{3b}$) obtenue à l'étape g2) de la concentration d'azote ammoniacal et d'AGV ($C_{3Ab}$, $C_{3AGVb}$) présents dans l'échantillon de substrat ou de digestat est compatible avec un modèle d'échantillon de substrat ou de digestat ($M_{Rb}$) associant lesdites concentrations d'azote ammoniacal et d'AGV ($C_{3Ab}$, $C_{3AGVb}$) à une fonction théorique de titrage de la capacité tampon $\beta$ ($\beta_{TCb}$) ;

- i2) On détermine des valeurs de pKa ($PKA_{Tb}$) correspondant aux points d'annulation de la dérivée de la fonction théorique de titrage de la dérivée de la conductivité par le pH ($DC_{T2b}$) associée au modèle de référence d'échantillon de substrat ou de digestat ($M_{Rb}$) qui présentent une erreur minimale ($E_{4minb}$) avec des valeurs de pKa ($PKA_{Eb}$) correspondants aux points d'annulation de la dérivée de la fonction expérimentale de titrage corrigée de la dérivée de la conductivité par le pH ($DC_{ECb}$) obtenue à l'étape d2) de manière à obtenir une estimation corrigée ($E_{4b}$) de la concentration d'azote ammoniacal et d'AGV ($C_{4Ab}$, $C_{4AGVb}$) présents dans ledit échantillon de substrat ou de digestat ;

- j2) On estime ($E_{5b}$) la concentration d'azote ammoniacal et d'AGV ($C_{5Ab}$, $C_{5AGVb}$) de la manière suivante :

  - lorsque l'estimation obtenue à l'étape g2) est compatible, l'estimation de la concentration d'azote ammoniacal et d'AGV correspond à la moyenne des estimations des concentrations d'azote ammoniacal et d'AGV des étapes g2) et i2) ;
  - lorsque l'estimation obtenue à l'étape g2) n'est pas compatible, l'estimation de la concentration d'azote ammoniacal et d'AGV correspond à l'estimation des concentrations d'azote ammoniacal et d'AGV de l'étape i2).

3. Procédé d'estimation selon la revendication 1 ou 2, **caractérisé en ce que** les données expérimentales ($D_{Ea}$, $D_{Eb}$) collectées sont soumises à au moins un traitement mathématique et à partir duquel on établit la fonction expérimentale de titrage ($\beta_{Ea}$, $DC_{Eb}$).

4. Procédé d'estimation selon la revendication 3, **caractérisé en ce que** ledit traitement mathématique consiste en au moins un traitement mathématique choisi parmi :

   - un lissage ;
   - un calcul de dérivée ;
   - un calcul de la capacité tampon $\beta$, ladite capacité tampon $\beta$ correspondant à la dérivée de la concentration de l'espèce titrante dans la solution réactionnelle en fonction du pH de la solution réactionnelle.

5. Procédé d'estimation de la concentration de composés d'intérêts choisis parmi le carbone inorganique, l'azote ammoniacal et les AGV présents dans un échantillon d'un substrat ou d'un digestat d'un méthaniseur, **caractérisé en ce qu'**on estime les concentrations de carbone inorganique, d'azote ammoniacal et d'AGV de la manière suivante :

   - la concentration de carbone inorganique est estimée selon le procédé d'estimation selon les revendications 1 et 3 à 4 ;
   - la concentration d'AGV et d'azote ammoniacal est estimée selon le procédé d'estimation selon les revendications 2 à 4.

6. Procédé d'estimation selon la revendication 5, **caractérisé en ce que** lorsqu'on effectue un titrage basique ($T_b$), suivi d'un titrage acide ($T_a$), alors on estime les concentrations de carbone inorganique, d'azote ammoniacal et d'AGV de la manière suivante :

   - la concentration de carbone inorganique est estimée selon le procédé d'estimation selon les revendications 1 et 3 à 4 ;
   - la concentration d'AGV et d'azote ammoniacal est estimée selon le procédé d'estimation selon les revendications 2 à 4.

**Patentansprüche**

1. Verfahren zum Abschätzen der Konzentration von bestimmten Bestandteilen, die aus dem anorganischen Kohlenstoff und den flüchtigen Fettsäuren (abgekürzt "VFA") ausgewählt sind, die in einer Probe eines Substrats oder eines Gärguts einer Biogasanlage vorhanden sind, **dadurch gekennzeichnet, dass** das Abschätzungsverfahren eine Säuretitration ($T_a$) durchführt und dass es mindestens die folgenden Schritte umfasst:

   a1) Verfügen über ein Referenzmodell einer Substrat- oder Gärgutprobe ($M_{Ra}$), das die Konzentration von anorganischem Kohlenstoff und von VFA ($C_{CIa}$, $C_{VFAa}$) dieses Referenzmodells einer Substrat- oder Gärgutprobe ($M_{Ra}$) mit einer theoretischen Titrationsfunktion der Pufferkapazität $\beta$ ($\beta_{Ta}$) verknüpft;
   b1) Erstellen einer experimentellen Titrationsfunktion der Pufferkapazität $\beta$ ($\beta_{Ea}$) ausgehend von experimentellen Daten ($D_{Ea}$), die während der Säuretitration ($T_a$) der Substrat- oder Gärgutprobe gesammelt wurden;
   c1) Identifizieren mittels des Referenzmodells einer Substrat- oder Gärgutprobe einer Konzentration von anorganischem Kohlenstoff und einer Konzentration von VFA ($C_{1CIa}$, $C_{1VFAa}$), für die die theoretische Titrationsfunktion der Pufferkapazität $\beta$ ($\beta_{Ta}$) dieses Referenzmodells einer Substrat- oder Gärgutprobe ($M_{Ra}$) einen minimalen Fehler ($E_{1mina}$) aufweist, mit der experimentellen Titrationsfunktion der Pufferkapazität $\beta$ ($\beta_{Ea}$), so dass die Konzentration von anorganischem Kohlenstoff und von VFA, die in der Substrat- oder Gärgutprobe ($C_{1CIa}$, $C_{1VFAa}$) vorhanden sind, abgeschätzt ($E_{1a}$) wird;
   d1) Überprüfen, dass die in Schritt c1) erhaltene Abschätzung ($E_{1a}$) der Konzentration von anorganischem Kohlenstoff und von VFA ($C_{1CIa}$, $C_{1VFAa}$), die in der Substrat- oder Gärgutprobe vorhanden sind, mit einem Referenzmodell einer Substrat- oder Gärgutprobe ($M_{Ra}$) kompatibel ist, das die Konzentrationen von anorganischem Kohlenstoff und von VFA ($C_{1CIa}$, $C_{1VFAa}$) mit einer theoretischen Funktion der Leitfähigkeit in Abhängigkeit vom pH ($C_{Ta}$) verknüpft;

e1) Eliminieren einer pH-Abweichung zwischen pH-Werten, die während der Säuretitration ($T_a$) gemessen wurden, und pH-Werten des Referenzmodells einer Substrat- oder Gärgutprobe ($M_{Ra}$), die ausgehend von den Konzentrationen von anorganischem Kohlenstoff und von VFA ($C_{1Cla}$, $C_{1VFAa}$) bestimmt wurden, die in Schritt c1) identifiziert wurden, um korrigierte experimentelle Daten ($D_{EC1a}$) zu erhalten, um ausgehend von diesen korrigieren experimentellen Daten ($D_{EC1a}$) eine korrigierte experimentelle Titrationsfunktion der Pufferkapazität β ($β_{EC1a}$) zu erstellen;

f1) Identifizieren, ausgehend von den korrigierten experimentellen Daten ($D_{EC1a}$), einer zweiten Konzentration von anorganischem Kohlenstoff und einer zweiten Konzentration von VFA ($C_{2Cla}$, $C_{2VFAa}$), für die eine theoretische Titrationsfunktion de Pufferkapazität β ($β_{T2a}$) des Referenzmodells einer Substrat- oder Gärgutprobe ($M_{Ra}$) einen minimalen Fehler ($E_{2mina}$) aufweist, mit der korrigierten experimentellen Titrationsfunktion der Pufferkapazität β ($β_{EC1a}$), um die korrigierte Konzentration von anorganischem Kohlenstoff und von VFA ($C_{2Cla}$, $C_{2VFAa}$), die in der Substrat- oder Gärgutprobe vorhanden sind, abzuschätzen $E_{2a}$;

g1) Bestimmen der Werte von pKa ($PKA_{Ta}$), die den Nullstellen der Ableitung der theoretischen Titrationsfunktion der Pufferkapazität β ($β_{T2a}$) des Referenzmodells einer Substrat- oder Gärgutprobe ($M_{Ra}$) entsprechen, die einen minimalen Fehler mit den pKa ($PKA_{Ea}$) aufweisen, die den Nullstellen der Ableitung der korrigierten experimentellen Titrationsfunktion der Pufferkapazität β ($β_{EC1a}$) entsprechen, um eine korrigierte Abschätzung ($E_{3a}$) der Konzentration von anorganischem Kohlenstoff und von VFA zu erhalten, die in der Substrat- oder Gärgutprobe ($C_{3Cla}$, $C_{3VFAa}$) vorhanden sind.

2. Verfahren zum Abschätzen der Konzentration von bestimmten Bestandteilen, die aus Ammoniumstickstoff und den VFA ausgewählt sind, die in einer Probe eines Substrats oder eines Gärguts einer Biogasanlage vorhanden sind, **dadurch gekennzeichnet, dass** das Abschätzungsverfahren eine Basetitration ($T_b$) durchführt und mindestens die folgenden Schritte umfasst:

- a2) Verfügen über ein Referenzmodell einer Substrat- oder Gärgutprobe ($M_{Rb}$), das die Konzentration von Ammoniumstickstoff und von VFA ($C_{Ab}$ und $C_{VFAb}$) dieses Referenzmodells einer Substrat- oder Gärgutprobe ($M_{Rb}$) mit einer theoretischen Titrationsfunktion der Ableitung der Leitfähigkeit durch den pH ($DC_{Tb}$) verknüpft;

- b2) Erstellen einer experimentellen Titrationsfunktion der Ableitung der Leitfähigkeit durch den pH ($DC_{Eb}$) ausgehend von experimentellen Daten ($D_{Eb}$), die während der Basetitration ($T_b$) der Substrat- oder Gärgutprobe gesammelt wurden;

- c2) Identifizieren mittels des Referenzmodells einer Substrat- oder Gärgutprobe ($M_{Rb}$) einer Konzentration von Ammoniumstickstoff und einer Konzentration von VFA ($C_{1Ab}$, $C_{1VFAb}$), für die die theoretische Titrationsfunktion der Ableitung der Leitfähigkeit durch den pH ($DC_{Tb}$) dieses Referenzmodells einer Substrat- oder Gärgutprobe ($M_{Rb}$) einen minimalen Fehler ($E_{1minb}$) aufweist, mit der experimentellen Titrationsfunktion der Ableitung der Leitfähigkeit durch den pH ($DC_{Eb}$), um die Konzentration von Ammoniumstickstoff und von VFA, die in der Substrat- oder Gärgutprobe ($C_{1Ab}$, $C_{1VFAb}$) vorhanden sind, abzuschätzen ($E_{1b}$);

- d2) Eliminieren einer pH-Abweichung zwischen den pH-Werten, die während der Basetitration ($T_b$) gemessen wurden, und den pH-Werten des Referenzmodells einer Substrat- oder Gärgutprobe ($M_{Rb}$), die ausgehend von den in Schritt c2) identifizierten Konzentrationen von Ammoniumstickstoff und von VFA ($C_{1Ab}$, $C_{1VFAb}$) bestimmt wurden, um korrigierte experimentelle Daten ($D_{EC1b}$) zu erhalten, um eine korrigierte experimentelle Titrationsfunktion der Ableitung der Leitfähigkeit durch den pH ($DC_{ECb}$) zu erstellen;

- e2) Identifizieren, ausgehend von den korrigierten experimentellen Daten ($DC_{ECb}$), einer zweiten Konzentration von Ammoniumstickstoff und einer zweiten Konzentration von VFA ($C_{2Ab}$, $C_{2VFAb}$), für die eine theoretische Titrationsfunktion der Ableitung der Leitfähigkeit durch den pH ($DC_{T2b}$) des Referenzmodells einer Substrat- oder Gärgutprobe ($M_{Rb}$) einen minimalen Fehler ($E_{2minb}$) aufweist, mit der korrigierten experimentellen Titrationsfunktion der Ableitung der Leitfähigkeit durch den pH ($DC_{ECb}$), um die korrigierte Konzentration von Ammoniumstickstoff und von VFA ($C_{2Ab}$, $C_{2VFAb}$), die in der Substrat- oder Gärgutprobe vorhanden sind, abzuschätzen ($E_{2b}$);

- f2) Erstellen, ausgehend von den korrigierten experimentellen Daten ($D_{EC1b}$), einer experimentellen Titrationsfunktion der Pufferkapazität β ($β_{Eb}$);

- g2) Identifizieren, mittels des Referenzmodells einer Substrat- oder Gärgutprobe ($M_{Rb}$), einer dritten Konzentration von Ammoniumstickstoff und einer dritten Konzentration von VFA ($C_{3Ab}$, $C_{3VFAb}$), für die eine theoretische Titrationsfunktion der Pufferkapazität β ($β_{Tb}$) des Referenzmodells einer Substrat- oder Gärgutprobe ($M_{Rb}$) einen minimalen Fehler ($E_{3minb}$) aufweist, mit der experimentellen Titrationsfunktion der Pufferkapazität β ($β_{Eb}$) aus Schritt f2), um die Konzentration von Ammoniumstickstoff und von VFA ($C_{3Ab}$, $C_{3VFAb}$), die in der Substrat- oder Gärgutprobe vorhanden sind, abzuschätzen;

- h2) Überprüfen, dass die in Schritt g2) erhaltene Abschätzung ($E_{3b}$) der Konzentration von Ammoniumstickstoff und von VFA ($C_{3Ab}$, $C_{3VFAb}$), die in der Substrat- oder Gärgutprobe vorhanden sind, mit einem Modell einer Substrat- oder Gärgutprobe ($M_{Rb}$) kompatibel ist, das die Konzentrationen von Ammoniumstickstoff und von VFA ($C_{3Ab}$, $C_{3VFAb}$) mit einer theoretischen Titrationsfunktion der Pufferkapazität $\beta$ ($\beta_{TCb}$) verknüpft;

- i2) Bestimmen der Werte von pKa ($PKA_{Tb}$), die den Nullstellen der Ableitung der theoretischen Titrationsfunktion von der Ableitung der Leitfähigkeit durch den pH ($DC_{T2b}$) entspricht, die mit dem Referenzmodell einer Substrat- oder Gärgutprobe ($M_{Rb}$) verknüpft ist, die einen minimalen Fehler ($E_{4minb}$) aufweisen, mit den Werten von pKa ($PKA_{Eb}$), die den Nullstellen der Ableitung der korrigierten experimentellen Titrationsfunktion der Leitfähigkeit durch den pH ($DC_{T2b}$) aus Schritt d2) entsprechen, um eine korrigierte Abschätzung ($E_{4b}$) der Konzentration von Ammoniumstickstoff und von VFA ($C_{4Ab}$, $C_{4VFAb}$) zu erhalten, die in der Substrat- oder Gärgutprobe vorhanden sind;

- j2) Abschätzen ($E_{5b}$) der Konzentration von Ammoniumstickstoff und von VFA ($C_{5Ab}$, $C_{5AGVb}$) folgendermaßen:

- wenn die Abschätzung aus Schritt g2) kompatibel ist, entspricht die Abschätzung der Konzentration von Ammoniumstickstoff und von VFA dem Mittelwert der Abschätzungen der Konzentrationen von Ammoniumstickstoff und von VFA der Schritte g2) und i2);
- wenn die Abschätzung aus Schritt g2) nicht kompatibel ist, entspricht die Abschätzung der Konzentration von Ammoniumstickstoff und von VFA der Abschätzung der Konzentrationen von Ammoniumstickstoff und von VFA von Schritt i2).

3. Abschätzungsverfahren nach Anspruch 1 oder 2, **dadurch gekennzeichnet, dass** die gesammelten experimentellen Daten ($D_{Ea}$, $D_{Eb}$) mindestens einer mathematischen Verarbeitung unterzogen werden, ausgehend von der die experimentelle Titrationsfunktion ($\beta_{Ea}$, $DC_{Eb}$) erstellt wird.

4. Abschätzungsverfahren nach Anspruch 3, **dadurch gekennzeichnet, dass** die mathematische Verarbeitung aus mindestens einer mathematischen Verarbeitung besteht, die ausgewählt ist aus:

- einer Glättung;
- einer Ableitungsberechnung;
- einer Berechnung der Pufferkapazität $\beta$, wobei die Pufferkapazität $\beta$ der Ableitung der Konzentration der titrierenden Spezies in der Reaktionslösung in Abhängigkeit vom pH der Reaktionslösung entspricht.

5. Verfahren zum Abschätzen der Konzentration von bestimmten Bestandteilen, die aus dem anorganischen Kohlenstoff, Ammoniumstickstoff und den VFA ausgewählt sind, die in einer Probe eines Substrats oder eines Gärguts einer Biogasanlage vorhanden sind, **dadurch gekennzeichnet, dass** die Konzentrationen von anorganischem Kohlenstoff, von Ammoniumstickstoff und von VFA folgendermaßen abgeschätzt werden:

- die Konzentration von anorganischem Kohlenstoff wird gemäß dem Abschätzungsverfahren nach den Ansprüchen 1 und 3 bis 4 abgeschätzt;
- die Konzentration von VFA und von Ammoniumstickstoff wird gemäß dem Abschätzungsverfahren nach den Ansprüchen 2 bis 4 abgeschätzt.

6. Abschätzungsverfahren nach Anspruch 5, **dadurch gekennzeichnet, dass**, wenn eine Basetitration ($T_b$), gefolgt von einer Säuretitration ($T_a$) durchgeführt wird, die Konzentrationen von anorganischem Kohlenstoff, von Ammoniumstickstoff und von VFA folgendermaßen abgeschätzt werden:

- die Konzentration von anorganischem Kohlenstoff wird gemäß dem Abschätzungsverfahren nach den Ansprüchen 1 und 3 bis 4 abgeschätzt;
- die Konzentration von VFA und von Ammoniumstickstoff wird gemäß dem Abschätzungsverfahren nach den Ansprüchen 2 bis 4 abgeschätzt.

## Claims

1. A method for estimating the concentration of compounds of interest selected from inorganic carbon and volatile fatty acids (abbreviated "VFA") present in a sample of a substrate or of a digestate from a methanizer, **characterized in that** said estimation method implements an acid titration ($T_a$) and that it comprises at least the following steps:

a1) A substrate or digestate sample reference model ($M_{Ra}$) associating the concentration of inorganic carbon and VFA ($C_{Cla}$ $C_{VFAa}$) of this substrate or digestate sample reference model ($M_{Ra}$) with a theoretical function of titration of the buffer capacity $\beta$ ($\beta_{Ta}$) is provided;

b1) An experimental function of titration of the buffer capacity $\beta$ ($\beta_{Ea}$) is established from experimental data ($D_{Ea}$) collected during said acid titration ($T_a$) of the substrate or digestate sample;

c1) Using the substrate or digestate sample reference model, an inorganic carbon concentration and a VFA concentration ($C_{1Cla}$, $C_{1VFAa}$) are identified for which the theoretical function of titration of the buffer capacity $\beta$ ($\beta_{Ta}$) of this substrate or digestate sample reference model ($M_{Ra}$) has a minimum error ($E_{1mina}$) with the experimental function of titration of the buffer capacity $\beta$ ($\beta_{Ea}$) so as to estimate ($E_{1a}$) the concentration of inorganic carbon and of VFA present in said substrate or digestate sample ($C_{1Cla}$, $C_{1VFAa}$);

d1) It is verified that the estimation ($E_{1a}$) obtained in step c1) of the concentration of inorganic carbon and of VFA ($C_{1Cla}$, $C_{1VFAa}$) present in the substrate or digestate sample is compatible with a substrate or digestate sample reference model ($M_{Ra}$) associating said inorganic carbon and VFA concentrations ($C_{1Cla}$, $C_{1VFAa}$) with a theoretical function of conductivity as a function of pH ($C_{Ta}$);

e1) A pH deviation between pH values measured during said acid titration ($T_a$) and pH values of the substrate or digestate sample reference model ($M_{Ra}$) determined from the inorganic carbon and VFA concentrations ($C_{1Cla}$, $C_{1VFAa}$) which have been identified in step c1) is removed so as to obtain corrected experimental data ($D_{EC1a}$) to establish from these corrected experimental data ($D_{EC1a}$) a corrected experimental titration function of the buffer capacity $\beta$ ($\beta_{EC1a}$);

f1) From said corrected experimental data ($D_{EC1a}$), a 2$^{nd}$ concentration of inorganic carbon and a 2$^{nd}$ concentration of VFA ($C_{2Cla}$, $C_{2VFAa}$) for which a theoretical function of titration of the buffer capacity $\beta$ ($\beta_{T2a}$) of the substrate or digestate sample reference model ($M_{Ra}$) has a minimum error ($E_{2mina}$) with the corrected experimental titration function for the buffer capacity $\beta$ ($\beta_{EC1a}$) are identified so as to estimate $E_{2a}$ the corrected concentration of inorganic carbon and of VFA ($C_{2Cla}$, $C_{2VFAa}$) present in said substrate or digestate sample.

g1) pKa values ($PKA_{Ta}$) corresponding to the cancellation points of the derivative of the theoretical titration function of the buffer capacity $\beta$ ($\beta_{T2a}$) of the substrate or digestate sample reference model ($M_{Ra}$) which have a minimal error with pKa ($PKA_{Ea}$) corresponding to the cancellation points of the derivative of the corrected experimental titration function of the buffer capacity $\beta$ ($\beta_{EC1a}$) are determined so as to obtain a corrected estimate ($E_{3a}$) of the concentration of inorganic carbon and of VFA present in said substrate or digestate sample ($C_{3Cla}$, $C_{3VFAa}$).

2. The method for estimating the concentration of compounds of interest selected from ammoniacal nitrogen and VFAs present in a sample of a substrate or of a digestate from a methanizer, **characterized in that** said estimation method implements a basic titration ($T_b$) and comprises at least the following steps:

- a2) A substrate or digestate sample reference model ($M_{Rb}$) associating the concentration of ammoniacal nitrogen and VFA ($C_{Ab}$ and $C_{VFAb}$) of this substrate or digestate sample reference model ($M_{Rb}$) with a theoretical function of titration of the derivative of the conductivity by pH ($DC_{Tb}$) is provided;

- b2) An experimental function of titration of the derivative of the conductivity by pH ($DC_{Eb}$) is established from experimental data ($D_{Eb}$) collected during said basic titration ($T_b$) of the substrate or digestate sample;

- c2) Using the substrate or digestate sample reference model ($M_{Rb}$), a concentration of ammoniacal nitrogen and a concentration of VFA ($C_{1Ab}$, $C_{1VFAb}$) for which the theoretical function of titration of the derivative of the conductivity by pH ($DC_{Tb}$) of this substrate or digestate sample reference model ($M_{Rb}$) has a minimal error ($E_{1minb}$) with the experimental function of titration of the derivative of the conductivity by pH ($DC_{Eb}$) are identified so as to estimate ($E_{1b}$) the concentration of ammoniacal nitrogen and VFA present in said substrate or digestate sample ($C_{1Ab}$, $C_{1VFAb}$);

- d2) A pH deviation between the pH values measured during said basic titration ($T_b$) and the pH values of the substrate or digestate sample reference model ($M_{Rb}$) determined from the concentrations of inorganic ammoniacal nitrogen and of VFA ($C_{1Ab}$, $C_{1VFAb}$) which have been identified in step c2) is removed so as to obtain corrected experimental data ($D_{EC1b}$) to establish a corrected experimental titration function of the derivative of the conductivity by pH ($DC_{ECb}$);

e2) From said corrected experimental data ($DC_{ECb}$), a 2$^{nd}$ concentration of ammoniacal nitrogen and a 2$^{nd}$ concentration of VFA ($C_{2Ab}$, $C_{2VFAb}$) for which a theoretical function of titration of the derivative of the conductivity by pH ($DC_{T2b}$) of the substrate or digestate sample reference model ($M_{Rb}$) has a minimum error ($E_{2minb}$) with the corrected experimental titration function of the derivative of the conductivity by pH ($DC_{ECb}$) are identified so as to estimate ($E_{2b}$) the corrected concentration of ammoniacal nitrogen and of VFA ($C_{2Ab}$, $C_{2VFAb}$) present in said substrate or digestate sample;

f2) From said corrected experimental data ($D_{EC1b}$), an experimental titration function of the buffer capacity $\beta$ ($\beta_{Eb}$) is established;

g2) Using the substrate or digestate sample reference model ($M_{Rb}$), a 3$^{rd}$ concentration of ammoniacal nitrogen

and a 3<sup>rd</sup> concentration of VFA ($C_{3Ab}$, $C_{3VFAb}$) for which a theoretical titration function of the buffer capacity β ($β_{Tb}$) of the substrate or digestate sample reference model ($M_{Rb}$) has a minimal error ($E_{3minb}$) with the experimental function of titration of the buffer capacity β ($β_{Eb}$) established in step f2) are identified so as to estimate the concentration of ammoniacal nitrogen and of VFA ($C_{3Ab}$, $C_{3VFAb}$) present in said substrate or digestate sample;

h2) It is verified that the estimate ($E_{3b}$) obtained in step g2) of the concentration of ammoniacal nitrogen and of VFA ($C_{3Ab}$, $C_{3VFAb}$) present in the substrate or digestate sample is compatible with a substrate or digestate sample reference model ($M_{Rb}$) associating said ammoniacal nitrogen and VFA concentrations ($C_{3Ab}$, $C_{3VFAb}$) with a theoretical titration function of the buffer capacity β ($β_{TCb}$);

i2) pKa values ($PKA_{Tb}$) corresponding to the cancellation points of the derivative of the theoretical titration function of the derivative of the conductivity by pH ($DC_{T2b}$) associated with the substrate or digestate sample reference model ($M_{Rb}$) which have a minimal error ($E_{4minb}$) with pKa values ($PKA_{Eb}$) corresponding to the cancellation points of the derivative of the corrected experimental titration function of the derivative of the conductivity by pH ($DC_{ECb}$) obtained in step d2) are determined so as to obtain a corrected estimate ($E_{4b}$) of the concentration of ammoniacal nitrogen and of VFA ($C_{4Ab}$, $C_{4VFAb}$) present in said substrate or digestate sample;

- j2) the concentration of ammoniacal nitrogen and of VFA ($C_{5Ab}$, $C_{5VFAb}$) is estimated ($E_{5b}$) as follows:

   - when the estimate obtained in step g2) is compatible, the estimate of the concentration of ammoniacal nitrogen and of VFA corresponds to the average of the estimates of the concentrations of ammoniacal nitrogen and of VFA of steps g2) and i2);
   - when the estimate obtained in step g2) is not compatible, the estimate of the concentration of ammoniacal nitrogen and of VFA corresponds to the estimate of the concentrations of ammoniacal nitrogen and of VFA of step i2).

**3.** The estimation method according to claim 1 or 2, **characterized in that** the collected experimental data ($D_{Ea}$, $D_{Eb}$) are subjected to at least one mathematical treatment and from which the experimental titration function ($β_{Ea}$, $DC_{Eb}$) is established.

**4.** The estimation method according to claim 3, **characterized in that** said mathematical treatment consists of at least one mathematical treatment selected from:

   - a smoothing;
   - a derivative calculation;
   - a calculation of the buffer capacity β, said buffer capacity β corresponding to the derivative of the concentration of the titrating species in the reaction solution as a function of the pH of the reaction solution.

**5.** A method for estimating the concentration of compounds of interest selected from inorganic carbon, ammoniacal nitrogen and VFAs present in a sample of a substrate or of a digestate from a methanizer, **characterized in that** the concentrations of inorganic carbon, of ammoniacal nitrogen and of VFAs are estimated as follows:

   - the inorganic carbon concentration is estimated according to the estimation method according to claims 1 and 3 to 4;
   - the concentration of VFA and of ammoniacal nitrogen is estimated according to the estimation method according to claims 2 to 4.

**6.** The estimation method according to claim 5, **characterized in that** when performing a basic titration ($T_b$), followed by an acid titration ($T_a$), then the concentrations of inorganic carbon, of ammoniacal nitrogen and of VFA are estimated as follows:

   - the inorganic carbon concentration is estimated according to the estimation method according to claims 1 and 3 to 4;
   - the concentration of VFA and of ammoniacal nitrogen is estimated according to the estimation method according to claims 2 to 4.

$M_{Ra}$ :

$C_{CIa}$ ; $C_{AGVa}$ $\longleftrightarrow$ $\beta_{Ta}$

a1)

$T_a \longrightarrow D_{Ea} \longrightarrow \beta_{Ea}$

b1)

$\beta_{Ea}$ ; $\beta_{Ta}$ $\Longrightarrow$ $E_{1mina}$

$E_{1a}$ : $C_{1CIa}$ ; $C_{1AGVa}$

c1)

$E_{1a}$ : $C_{1CIa}$ ; $C_{1AGVa}$ $\longrightarrow$ $M_{Ra}$ $\longleftrightarrow$ $C_{Ta}$

d1)

$D_{EC1a} \longrightarrow \beta_{EC1a}$

e1)

$\beta_{EC1a}$ ; $\beta_{T2a}$ $\Longrightarrow$ $E_{2mina}$

$E_{2a}$ : $C_{2CIa}$ ; $C_{2AGVa}$

f1)

$PKA_{Ta}$ ; $PKA_{Ea}$ $\Longrightarrow$ $E_{3mina}$

$E_{3a}$ : $C_{3CIa}$ ; $C_{3AGVa}$

g1)

**FIG. 1**

$M_{Rb}$ :

$C_{Ab}$ ; $C_{AGVb}$  ⟷  $DC_{Tb}$ — a2)

$T_h$ ⟶ $D_{Fh}$ ⟶ $DC_{Fh}$ — b2)

$DC_{Tb}$ ; $DC_{Eb}$  ⟹  $E_{1minb}$ — C2)

$E_{1b}$ : $C_{1Ab}$ ; $C_{1AGVb}$

$D_{EC1b}$ ⟶ $DC_{ECb}$ — d2)

$DC_{ECb}$ ; $DC_{T2b}$  ⟹  $E_{2minb}$ — e2)

$E_{2b}$ : $C_{2Ab}$ ; $C_{2AGVb}$

$D_{EC1b}$ ⟶ $\beta_{Eb}$ — f2)

$\beta_{Eb}$ ; $\beta_{Tb}$  ⟹  $E_{3minb}$ — g2)

$E_{3b}$ : $C_{3Ab}$ ; $C_{3AGVb}$

$E_{3b}$ : $C_{3Ab}$ ; $C_{3AGVb}$ ⟶ $M_{Rb}$ ⟷ $\beta_{TCb}$ — h2)

$PKA_{Tb}$ ; $PKA_{Eb}$  ⟹  $E_{4minb}$ — i2)

$E_{4b}$ : $C_{4Ab}$ ; $C_{4AGVb}$

$E_{5b}$ : $C_{5Ab}$ ; $C_{5AGVb}$ = MOY ( $E_{3b}$; $E_{4b}$) — j2)

**FIG. 2**

Fig. 3

Fig. 4

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP H05169100 A

- US 5640330 A